# EUROPEAN PATENT APPLICATION

(11) **EP 1 398 371 A1**
(43) Date of publication of application: **17.03.2004**
(21) Application number: 02292254.6
(22) Date of filing: 13.09.2002
(51) Int. Cl.: C12N 7/04, C12N 15/51, C12N 15/86, A61K 39/29, A61K 48/00, G01N 33/569, C12Q 1/70

(54) **Infectious HCV pseudo-particles containing functional E1, E2 envelope proteins**

(71) Applicant: INSTITUT NATIONAL DE LA SANTE ET DE LA RECHERCHE MEDICALE (INSERM), 75013 Paris (FR)
(72) Inventor: Bartosch, Birke, 69630 Simandres (FR); Cosset, François-Loic, 69007 Lyon (FR)
(74) Representative: Bernasconi, Jean Raymond

(57) **Abstract**

The invention relates to the generation and the use of HCV pseudo-particles containing native functional E1, E2 envelope glycoproteins assembled onto retroviral core particles. These particles are highly infectious and constitute a valid model of HCV virion.

## Description

The invention relates to the generation and the use of HCV pseudo-particles containing native functional E1, E2 envelope glycoproteins assembled onto retroviral core particles. These particles are highly infectious and constitute a valid model of HCV virion.

World-wide several hundred millions of people are infected with hepatitis C virus (HCV) (Lavanchy et al., 1999). Progression to chronic disease occurs in the majority of HCV infected persons. Infection is associated with an increased risk for liver diseases and hepato-cellular carcinoma and has become the main indication for liver transplantation. HCV infection also increases the number of complications in HIV infected people (Dieterich, 2002). No vaccine is currently available to prevent new infections and the only treatment for chronic hepatitis C is interferon-α therapy, either alone or in combination with the guanosine analogue ribavirin. However, only ~40% of patients respond to treatment. Clearly, novel therapeutic strategies are urgently required as the health costs for HCV infected people are predicted to spiral dramatically in the next few decades.

HCV proteins structural and non structural proteins are expressed from a single polyprotein precursor and individually released in their respective cell compartments upon cleavage by cellular and viral proteases (Lindenbach and Rice, 2001). By analogy with other members of the Flaviviridae, HCV genomic organization suggests a virus consisting of a nucleocapsid comprising a viral genome and core protein (C) coated by a lipid envelop containing the two envelope glycoproteins E1 and E2.

So far, the study of hepatitis C virus (HCV), has been hampered by the low level of HCV particles in infected patients and by the lack of available efficient and reliable culture system for amplifying the virus (Lindenbach and Rice, 2001). Recently, a model for HCV replication, based on the self-replication of engineered minigenomes in cell culture, has been established (Blight et *al.*, 2000; Lohmann *et al.,* 1999). Although very useful to study HCV genomic replication, this system does not support production of HCV particles (Pietschmann *et al.,* 2002).

Production of HCV virus-like particles (VLP) in insect cells has already been reported (WO 98/21338, Wellnitz et *al*., 2002; Baumert et *al*.; 1998, Owsianka et *al.,* 2001). However these particles were not secreted and their extraction from intracellular compartments yielded VLP preparations that were not infectious. Pseudotyped Vesicular Stomatitis Virus (VSV) viral particles have also been engineered with chimeric E1 and/or E2 glycoproteins whose transmembrane domains were modified to allow their transport to the cell surface (Buonocore et *al.,* 2002; Matsuura et *al.,* 2001). However, such modifications are likely to disturb conformation and functions of the E1E2 complexes (Matsuura et *al.,* 2001) and although such pseudo-particles stand among candidate HCV vaccines (Rose et *al.,* 2001), their use as a tool to investigate HCV assembly and cell entry remains controversial (Buonocore et *al.,* 2002). Chimeric HCV-BVDV (Bovine Viral Diarrhea Virus) particles were shown to be infectious for the human hepatocyte line Huh-7 (WO 00/75352). However, infectivity could not be neutralised by an anti-serum against HCV which indicated that these particles did not constitute a valid model of HCV virion.

New approaches are therefore sorely needed to study HCV assembly and cell entry in order to design HCV cell entry inhibitors and to study the humoral immune response against HCV. Availability of infectious, amplifiable HCV particles would also provide useful material for the development of diagnostic application as well as therapeutical drugs.

The invention thus overcomes these hurdles by proposing infectious, HCV pseudo-particles harboring unmodified E1 and E2 glycoproteins.

### Definitions

The terms *"vector",* "*cloning vector"* and "*expression vector"* mean the vehicle by which a DNA or RNA sequence (e.g. a foreign gene) can be introduced into a host cell, so as to transform the host and promote expression (e.g. transcription and translation) of the introduced sequence. Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a *"DNA construct*". A common type of vector is a *"plasmid",* which generally is a self-contained molecule of double-stranded DNA, usually of bacterial origin, that can readily accept additional (foreign) DNA and which can readily be introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmid and fungal vectors, have been described for replication and/or expression in a variety of eukaryotic and prokaryotic hosts.

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme.

The term "transfection" means the introduction of a foreign nucleic acid (DNA, cDNA or RNA) into a cell so that the host cell will express the introduced gene or sequence to produce a desired substance, typically a protein coded by the introduced gene or sequence. The introduced gene may include regulatory or control sequences, such as start, stop, promoter, signal, secretion, or other sequences used by a cell's genetic machinery. A host cell that receives and expresses introduced DNA or RNA has been *"transformed".*

The term *"host cell"* means any cell of any organism that is selected, modified, transformed, grown, or used or manipulated in any way, for the production of a substance by the cell, for example the expression by the cell of a gene, a DNA sequence, a protein, a virion. In the context of the invention, the host cell is a mammalian cell. Suitable host cells include Huh-7 human hepatocellular carcinoma (Nakabayashi et *al.,* 1982), Hep3B human hepatocellular carcinoma (ATCC HB-8064), HepG2 human hepatocellular carcinoma (HB-8065), HT-1080 human fibrosarcoma (CCL-*121*), 293T human embryo kidney cells (ATCC CRL-1573), TE671 human rhabdomyosarcoma (ATCC CRL-8805), Jurkat human T cell leukemia (TIB-152), CEM human lymphoblastic leukemia (CCL-119), COS-7 African green monkey fibroblasts kidney (CRL-1651 ), VERO African green monkey kidney (CCL-81 ), PG-4 feline astrocyte (CRL-2032), BHK-21 golden hamster kidney (CCL-10), CHO Chinese hamster ovary (ATCC CCL-61), and NIH3T3 mouse fibroblasts. In a specific embodiment said host cell is 293T.

As used herein, the term *"permissive cell"* is meant for a cell that is permissive for HCV infection.

*"Hepatitis C Virus"* or *"HCV"* is a member of the flavivirus family. HCV genome encodes a single polyprotein NH₂-C-E1-E2-NS2-NS3-NS4a-NS4b-NS5a-NS5b-COOH that is processed co and post-translationally into both structural (N-terminal nucleocapsid protein termed "Core" (C), and glycoproteins E1 and E2) and non-structural (NS) proteins. In the context of the invention, HCV is of any genotype, *e.g.* 1, 2, 3, 4, 5, 6, and subtype, e.g. a, b, c, d, e, f, g, h, k, and variants thereof.

The term *"variant"* refers to the homologous polynucleotide sequences and corresponding amino acid sequences found in the different HCV strains owing to HCV hypervariability.

Preferably, HCV has either 1a or 1b genotype (Dubuisson *et al.,* 1994) that stand among HCV genotypes that are the most prevalent and the most resistant to interferon-α therapy (Zein, 2000).

The term *"HCV-like particles"* or *"HCV pseudo-particles"* as used herein refers to non naturally occurring viral particles that comprise an envelope protein of HCV. The HCV pseudo-particles of the invention are infectious for a target cell. The particles of the invention more particularly comprise retroviral core proteins. Such particles may be readily produced by one skilled in genetic engineering techniques. One can for instance refer to EP 1 201 750 that describes production of synthetic retroviral particles expressing an antigen for modulating an immune response.

In the context of the invention, the term "*infectious*" is used to describe the capacity of the particles of the invention to complete the initial steps of viral cycle that lead to cell entry. However, upon interaction with the host cell, HCV-like particles may or may not produce progeny viruses.

The term *"an envelope protein of HCV"* denotes the E1 or E2 glycoprotein of HCV, or a mutant thereof.

By an "*E1 glycoprotein*" or "*E1 protein*" is meant a native envelope 1 protein (E1) from any genotype, subtype and variant of HCV strains.

By an "*E2 glycoprotein*" or "*E2 protein*" is meant a native envelope 2 protein (E2) from any genotype subtype and variant of HCV strains.

Preferably, E1 and E2 glycoproteins are derived from a same HCV strain.

The term *"mutanf'* or "*mutation*" is meant for alteration of the DNA sequence that result in a modification of the amino acid sequence of native E1 or E2 proteins. Such a modification can be for instance the substitution and/or deletion of one or more amino acids. Mutants notably include fragments of native E1 and E2 proteins. Variants are particular examples of naturally occurring mutants. Mutants are more particularly contemplated as useful for identifying the structural elements of E1 and/or E2 proteins necessary for maintaining cell infectivity or for increasing E1 and/or E2 antigenicity for vaccination purposes. In a preferred embodiment, the mutants encompass E2 glycoproteins wherein hypervariable region I has been deleted, while the particles produced therefrom remain infectious.

As used herein, the term "*HCV core*" denotes a native core protein of the various HCV strains, a fragment thereof, or a variant thereof. The HCV core provides a signal peptide for the E1 or optionally E2 linked thereto that allows protein translocation to the endoplasmic reticulum. HCV core signal peptide correspond to the last 21 residues of the carboxy-terminus of HCV core (GCSFSIFLLALLSCLTVPASA, SEQ ID N°1). According to an embodiment, the core protein is thus a N-terminally truncated form of HCV core (ΔC). Preferably ΔC comprises the last 21 residues of the carboxy-terminus of HCV core. In particular, HCV core may consist in the last 60 residues of the carboxy-terminus of HCV core.

In the context of the invention, the terms *"native"* or *"unmodified"* are indifferently used to describe a wild-type, full-length protein.

The term *"polyprotein"* as used herein is used to describe a protein construct made up of individual proteins that are joined together in a sequence whereby they retain their original relevant biological activities.

The term *"a polyprotein comprising a HCV core protein linked to HCV E1 protein and*/*or HCV E2 protein*" includes the CE1E2, CE2E1, CE1, CE2, ΔCE1E2, ΔCE1, and ΔCE2 polyproteins. *"CE1E2"* denotes a polyprotein comprising successively a HCV core protein, a HCV E1 protein and a HCV E2 protein. *"CE2E1"* denotes a polyprotein comprising successively a HCV core protein, a HCV E2 protein and a HCV E1 protein. *"CE1"* denotes a polyprotein comprising a HCV core protein linked to a HCV E1 protein. *"CE2"* denotes a polyprotein comprising a HCV core protein linked to a HCV E2 protein. *"ΔCE1E2"* denotes a polyprotein comprising a carboxy terminus of HCV core protein, and HCV E1 and HCV E2 proteins. *"ΔCE1"* denotes a polyprotein comprising a carboxy terminus of HCV core protein, and a HCV E1 protein. *"ΔCE2"* denotes a polyprotein comprising a carboxy terminus of HCV core protein, and a HCV E2 protein. *ΔCE1E2,* as well as *ΔCE2,* have been built by inserting a stop codon at the end of E2, whereas *ΔCE1* has been built by inserting a stop codon at the end of E1.

By "*retrovirus*" is meant a virus whose genome consists of a RNA molecule and that comprises a reverse-transcriptase, *i.e.* a member of the Retroviridae family. Retroviruses are divided into Oncovirus, Lentivirus and Spumavirus. Preferably said retrovirus is an oncovirus, *e.g.* MLV, ALV, RSV, or MPMV, a lentivirus, e.g. HIV-1, HIV-2, SIV, EIAV, or CAEV, or a spumavirus such as HFV. Genomes of these retroviruses are readily available in databanks.

In the context of the invention "a *nucleic sequence comprising a packaging competent retrovirus-derived genome*" is intended for a sequence that comprises the retroviral nucleic acid sequences known as "cis-acting" sequences. These include the Long Terminal Repeats (LTRs) for the control of transcription and integration, the psi sequence necessary for encapsidation, and the Primer Binding site (PBS) and polypurine track (PPT) sequences necessary for reverse transcription of the retroviral genome. Advantageously, said nucleic acid sequence comprising a packaging competent retrovirus-derived genome further comprises a transgene.

Said retroviral genome may be replication-defective or replication-competent, in the absence of any trans-complementing function. A replication-competent genome would further comprise the gag, pol, and env retroviral genes. In a replication-defective genome, the viral genes gag, pol, and env are deleted. However, assembly of viral pseudo-particles may be achieved by providing another vector that comprises gag, pol and env but that is defective for the "cis" sequences. Their expression allows the encapsidation of the transgene, excluding the genes necessary for the multiplication of the viral genome and for the formation of complete viral particles.

As used herein, the term "transgene" designates the gene that is expressed in the target cell upon infection by the particles of the invention.

Examples of transgenes include a gene encoding a molecule of therapeutic interest, a marker gene, a gene coding for an immune modulator, an antigen, or a suicide gene.

A *"marker gene"* denotes a gene whose expression is detectable. For instance marker gene expression can generate a detectable signal, such as a fluorescence emission, a chromogenic reaction, or confer a growth advantage to the cells wherein it is expressed (antibiotic resistance genes).

An *"immune modulator"* refers to the product of a gene that modifies the activity of the immune system of a subject *in vivo.* Examples of immune modulators include cytokines, *(e.g.* interleukins, interferons, or haematopoietic colony stimulating factors), chemokines, and the like. Expression of an immune modulator by transformed cells may change the cellular environment and alter differentiation of immune cells and thus modify the type and the strength of immune response elicited against a given antigen.

An "*antigen*" refers to a molecule, such as a peptide, a polypeptide or a protein, against which an immune response is sought. Said antigen may be for instance a tumor, a bacterial, a pathogenic, a proteic, or a viral antigen.

A "*suicide gene*" is meant for a gene whose expression in cells induces programmed-cell death (apoptosis) such as the conditional Herpes Simplex virus type I thymidine kinase gene.

The "*core protein from a retrovirus*" refers to proteins encoded by the gag and pol genes. The gag gene encodes a polyprotein which is further processed by the retroviral protease into structural proteins that comprise the core. The pol gene encodes the retroviral protease, reverse-transcriptase, and integrase.

A *"pharmaceutically acceptable carrier"* refers to any vehicle wherein the vaccine composition according to the invention may be formulated. It includes a saline solution such as phosphate buffer saline. In general, a diluent or carrier is selected on the basis of the mode and route of administration, and standard pharmaceutical practice.

In the context of the present application, "*vaccination*" is intended for prophylactic or therapeutical vaccination. *"Therapeutical vaccination"* is meant for vaccination of a patient with HCV infection.

According to the invention, the term *"subject"* or *"patient"* is meant for any mammal likely to be infected with HCV. Human, chimpanzee, tamarin, and mice, especially human liver-xenogratfed mice are examples of hosts for HCV.

### Production of HCV particles

The inventors have generated infectious pseudo-particles that contain functional, and more particularly unmodified, HCV glycoproteins assembled onto retroviral core particles. HCV E1 E2 were expressed from a polyprotein containing the core (C) protein or a fragment thereof, in particular the carboxy-terminus of the C protein, which served as signal peptide for E1 or E2, and the E1 and/or E2 glycoproteins.

The invention thus provides a method for producing hepatitis C virus (HCV)-like particles *ex vivo* comprising the steps of:
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a HCV core protein, and a HCV E1 protein and/or a HCV E2 protein;
- transfecting host cells with said nucleic acid sequences and maintaining the transfected cells in culture for sufficient time to allow expression of the cDNAs to produce structural proteins from hepatitis C virus and retrovirus; and allowing the structural proteins to form virus-like particles.

The invention further provides a method for producing hepatitis C-virus (HCV)-like particles *in vivo,* which method comprises the steps of :
- providing a first nucleic acid sequence comprising a packaging competent retrovirus-derived genome;
- providing a second nucleic acid sequence comprising a cDNA encoding the core proteins from said retrovirus;
- providing a third nucleic acid sequence comprising a cDNA encoding a polyprotein comprising successively a HCV core protein, and a HCV E1 protein and/or a HCV E2 protein;
- transfecting cells of a subject *in vivo* with said nucleic acid sequences, to allow expression of the cDNAs to produce structural proteins from hepatitis C virus and retrovirus; and to allow the structural proteins to form virus-like particles.

Another aspect of the invention is the use of three nucleic acid sequences for the preparation of a medicament useful as a vaccine against hepatitis C, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a HCV core protein, and a HCV E1 protein and/or a HCV E2 protein ;
and, when transferred into cells of a subject, the nucleic acid sequences allow the production of structural proteins from hepatitis C virus and retrovirus, wherein the structural proteins form virus-like particles that are immunogenic.

For the purpose of transfection, said first, second and third nucleic acid sequences may be carried on a same vector, or on two or three separated vectors.

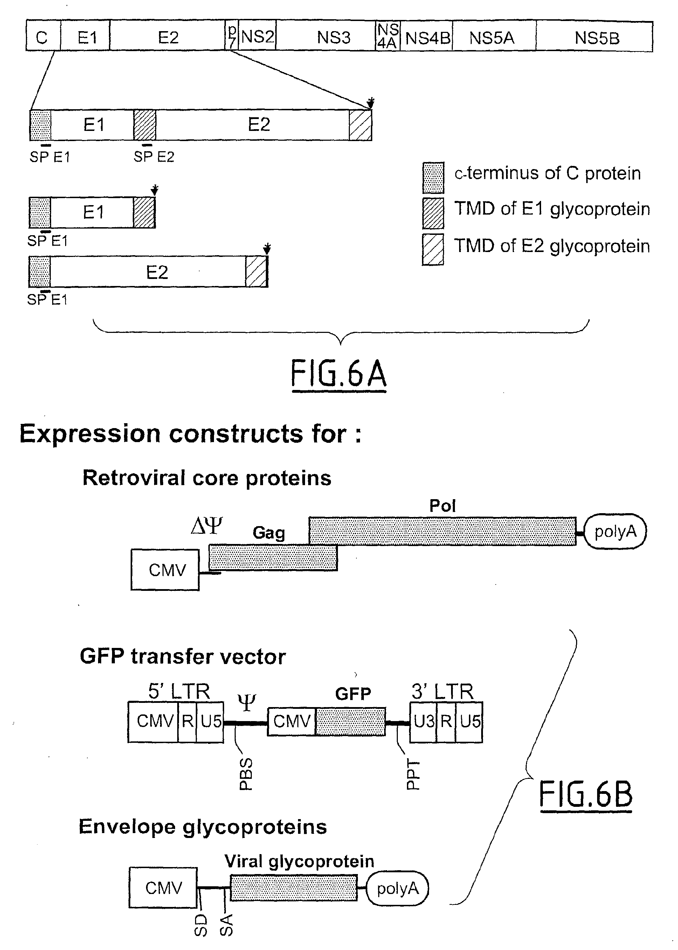

An example of HCV E1 E2 and retroviral expression constructs is shown in figures 6A and 6B.

According to a specific embodiment, said packaging competent retroviral genome and core proteins are derived from a retrovirus selected from the group consisting of MLV, ALV, RSV, MPMV, HIV-1, HIV-2, SIV, EIAV, CAEV, and HFV.

Advantageously, the packaging competent retroviral genome further comprises a marker gene or an immune modulator.

In the method of the invention, said polyprotein may comprise a HCV core protein linked to a HCV E1 protein, a HCV core protein linked to a HCV E2 protein, or successively a HCV core protein, a HCV E1 protein and a HCV E2 protein, or successively a HCV core protein, a HCV E2 protein and a HCV E1 protein.

According to an embodiment, E1 and/or E2 are native proteins. According to another embodiment, E1 and/or E2 glycoproteins are mutated to obtain particles that are useful for characterizing the glycoprotein determinants for HCV infectivity.

Preferably, said E1 and E2 glycoproteins are both derived from a same HCV strain.

According to another embodiment said HCV core protein is a carboxy terminus form (ΔC) of HCV core protein. In particular, said HCV core protein may comprise the last 21 amino acids of the carboxy-terminus of HCV core.

In accordance with the present invention there may be employed conventional molecular biology, microbiology, and recombinant DNA techniques within the skill of the art. Such techniques are explained fully in the literature. See, *e.g.*, Sambrook et *al.*, 1989 ; DNA Cloning: A Practical Approach, Volumes I and II (D.N. Glover ed. 1985) ; Oligonucleotide Synthesis (M.J. Gait ed. 1984) ; Nucleic Acid Hybridization [B.D. Hames & S.J. Higgins eds. (1985)] ; Transcription and Translation [B.D. Hames & S.J. Higgins, eds. (1984)] ; Animal Cell Culture [R.I. Freshney, ed. (1986)] ; Immobilized Cells and Enzymes [IRL Press, (1986)] ; B. Perbal, A Practical Guide To Molecular Cloning (1984) ; F.M. Ausubel et *al.,* 1994.

In particular, the vectors of the invention may be introduced into the target cell by means of any technique known for the delivery of nucleic acids to the nucleus of cells, either in culture, *ex vivo,* or *in vivo.*

Introduction of the nucleic acid sequences may be performed by any standard method well known by one skilled in the art, *e.g.* transfection, electroporation, microinjection, transduction, cell fusion, DEAE dextran, calcium phosphate precipitation, or use of a gene gun (see for instance Wu et al., 1992 ; Wu et al, 1988).

The donor nucleic acid targeting system can also be introduced by lipofection. In certain embodiments, the use of liposomes and/or nanoparticles is contemplated for the introduction of the donor nucleic acid targeting system into host cells. Nanocapsules can generally entrap compounds in a stable and reproducible way. Ultrafine particles (sized around 0.1 µm) that can be designed using biodegradable polyalkyl-cyanoacrylate polymers are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core. The use of cationic lipids may promote encapsulation of negatively charged nucleic acids, and also promote fusion with negatively charged cell membranes (Felgner et al., 1989).

*In vivo* targeted gene delivery is described in international patent publication WO 95/28 494. Alternatively, the vector can be introduced *in vivo* by lipofection, using liposomes or nanoparticles as above described. It is also possible to introduce the vector *in vivo* using techniques that are similar to the techniques that are employed *in vitro* (e.g. transfection, electroporation...).

### HCV-like particles

In the method described above no structural modifications of the E1 E2 glycoproteins are required for their correct assembly on retroviral cores. The method of the invention thus makes it possible to generate high titre infectious HCV pseudo-particles with functional E1E2 glycoproteins. As demonstrated herein, these particles constitute a valid model of HCV virion as regards to early steps of viral infection cycle.

The invention further relates to an infectious HCV-like particle, comprising the core proteins from a retrovirus, and E1 and/or E2 HCV glycoprotein(s). Such a particle is obtainable by a method as described above.

According to an embodiment, the infectious particle of the invention may comprise native HCV E1 protein, native HCV E2 protein, or native HCV E1 protein and native HCV E2 protein. Preferably said E1 and E2 glycoproteins are both derived from a same HCV strain. According to another embodiment, E1 and/or E2 glycoproteins are mutated.

In a particular embodiment, the hypervariable region I (located in the N-terminus region, *i.e,* the first 27 aminoacids of the E2 protein after the signal peptide) is deleted. The HCV-like particles (called ΔHVR1) produced with E2 protein deleted from this region are particularly advantageous as a diagnostic tool or in vaccination, to enhance induction or binding of neutralizing antibodies. The ΔHVR1 HCV-like particles are also of interest to identify the epitopes/mechanisms within the HCV glycoproteins that can be targeted/inhibited by neutralizing antibodies or other therapeutic agents.

Said retrovirus may be selected from the group consisting of MLV, ALV, RSV, MPMV, HIV-1, HIV-2, SIV, EIAV, CAEV, and HFV.

Advantageously, said infectious particles further carry a transgene. For instance said transgene may be a marker gene which make it possible to follow-up cell infection by the infectious particles of the invention and can find application for instance in the identification of a cell receptor involved in HCV entry. Said transgene can also be a gene encoding a molecule of therapeutic interest and/or a suicide gene. Accordingly, the particles of the invention that specifically target primary or cancerous hepatocytes comprise a useful vector for gene transfer and/or gene therapy.

### Use of the infectious HCV-like particles of the invention

High infectivity of these particles makes it possible for the investigation of the role of HCV E1 and E2 glycoproteins and their potential receptors in cell entry, HCV host-range and neutralisation by antibodies from HCV patient sera.

The invention therefore concerns the use of an HCV-like infectious particle as described above, for *ex vivo* identification of a cell receptor for HCV E1 and/or E2 glycoprotein.

According to an embodiment, the invention provides a method for ex vivo identification of a receptor for HCV E1 and/or E2 glycoprotein comprising detection of the binding of said particle to a cell receptor. More specifically, the method may comprise the steps consisting of:
- contacting a cell susceptible to HCV infection with an infectious HCV-like particle of the invention, under conditions sufficient to allow specific binding of said particle to a receptor expressed at the surface of said cell;
- detecting binding of said particle to a receptor; and
- identifying said receptor.

A cell susceptible to HCV infection may preferably be selected from the group consisting of a hepatocyte cell line, such as Huh-7 human hepatocellular carcinoma (Nakabayashi et *al.,* 1982), Hep3B human hepatocellular carcinoma (ATCC HB-8064), or HepG2 human hepatocellular carcinoma (HB-8065), and a primary human hepatocyte. Primary human hepatocytes may be isolated from human adult biopsy samples according to procedures well-known by one skilled in the art. One can for example refer to Guguen-Guillouzo and Guillouzo (1986). Otherwise such cells are commercially available, and can be purchased for instance from Biopredic International (Rennes, France).

Detection of particle binding to a receptor can be achieved according to classical procedures well-known by one skilled in the art. For instance, this could involve radioactive, enzyme or fluorescent labelling of the particles of the invention, and subsequent detection with an appropriate method. A number of fluorescent materials are known and can be utilized as labels. These include, for example, fluorescein, rhodamine, auramine, Texas Red. Enzyme labels consist in conjugation of an enzyme to a molecule of interest, *e.g.* a polypeptide, and can be detected by any of colorimetric, spectrophotometric, or fluorospectrophotometric techniques. Flow cytometry analysis (FACS) together with labelled antibodies directed against E1 or E2 proteins harboured by the pseudo-particles of the invention is also appropriate.

According to another embodiment, the invention provides a method for *ex vivo* identifying a cell receptor for HCV comprising the step consisting of:
- transfecting a cell which is not permissive for HCV infection with a nucleic acid sequence encoding a protein likely to be a receptor for HCV;
- contacting said transformed cell with a HCV-like particle of the invention;
- determining whether said transformed cell has become permissive or not for HCV infection; and
- identifying as a cell receptor for HCV said protein expressed by the transformed cell that has become permissive.

Determination of whether the transformed cell has become permissive for HCV infection can be readily achieved using the HCV-like particles of the invention. In particular, where said particles carry a marker gene, such as GFP, permissivity (i.e. the capacity of cells to be infected with HCV, and in particular with HCV-like particles) can be assessed by FACS analysis of the transformed cells. Where the marker gene is an antibiotic resistance gene, identification of cells infected by HCV-like particle is readily achieved through exposure to said antibiotic.

Where one do not suspect a given protein to be a receptor for HCV entry in cells, the above method can advantageously be adapted for the screening and the identification of a cell receptor for HCV. In particular, an expression cDNA library can be prepared, for instance from a cDNA library obtained by reverse-transcription of cellular mRNAs from a cell permissive for HCV infection. Expression of such a cDNA library would be driven by a constitutive promoter whose nucleic acid sequence has been fused to the cDNA library in suitable vectors. Such a library would contain a vector encoding a cell receptor for HCV. Non permissive cells can then be transfected with this expression library and further screened for the identification of a cell receptor for HCV.

To this end, the invention proposes a method for *ex vivo* identifying a cell receptor for HCV comprising the step consisting of:
- providing an expression cDNA library obtained from a cell permissive for HCV infection;
- transfecting cells that are not permissive for HCV infection with said expression cDNA library;
- contacting said transformed cells with HCV-like particles of the invention;
- identifying and isolating those transformed cells that have become permissive for HCV infection;
- isolating the expression vector transfected in cells that have become permissive; and
- identifying as a receptor for HCV the proteins encoded by the cDNA sequence of said isolated expression vectors.

Determination of whether the transformed cell has become permissive for HCV infection can be readily achieved using the HCV-like particles of the invention. In particular, where said particles carry a marker gene, such as GFP, permissivity *(i.e.* the capacity of cells to be infected with HCV, and in particular with HCV-like particles) can be assessed by FACS analysis of the transformed cells. Where the marker gene is an antibiotic resistance gene, identification of cells infected by HCV-like particle is readily achieved through exposure to said antibiotic.

Advantageously, the expression cDNA library is expressed from retroviral vectors that comprises glycoproteins that allow infection of the HCV non permissive cells. Such glycoproteins can be the VSV-G glycoprotein derived from vesicular stomatitis virus (VSV) whose receptor is expressed in most cell types ex *vivo.* Such viral particles can be assembled using a packaging competent retrovirus-derived genome that comprises the expression cDNA library, and optionally a marker gene. According to this embodiment the method for isolating the expression vector expressed in cells that have become permissive to infection by the HCV-like particles of the invention is greatly facilitated. Indeed this latter embodiment is particularly advantageous in that the process of cell infection with retroviral vectors has greater efficacy, as compared to cell transfection. Furthermore, cell infection leads to stable integration of viral genome in the cellular genome. Accordingly, transgenes, *i.e.* cDNA and marker gene that are carried by the pseudo-particles of the invention, are found to be stably expressed by infected cells. This in contrast with classical vectors used for transfection that do not integrate into cellular genome and for which expression may be transient.

In another aspect, the invention relates to the use of an infectious particle as defined above, for the identification of molecules capable of interfering with HCV entry in cells. In particular, herein is provided a method of *ex vivo* screening or identification of molecules capable of interfering with HCV entry in cells comprising comparison of the level of cell infection by the particles of the invention in the presence or the absence of a candidate molecule. Said method preferably comprises the steps consisting of:
- contacting a cell susceptible to HCV infection with an infectious HCV-like particle, in the absence or presence of a candidate molecule, under conditions that allow cell infection with HCV-like particle in the absence of any candidate molecule;
- assessing cell infectivity in the absence and in the presence of said candidate molecule;
- comparing cell infectivity measured in presence of said candidate molecule with cell infectivity measured in absence of any candidate molecule;
- identifying as a molecule capable of interfering with HCV entry the candidate molecule for which cell infectivity, as measured in the presence of said molecule, is decreased as compared to cell infectivity measured in the absence of any candidate molecule.

Contacting a cell susceptible to HCV infection with an infectious HCV-like particle, and a candidate molecule can be carried out by contacting simultaneously said cell, HCV-like particle and candidate molecule. Otherwise, two of these three elements can be contacted under conditions sufficient to allow their interaction before addition of the third missing element.

Cell infectivity can be readily assessed by one skilled in the art. One can take advantage of the embodiment wherein the infectious HCV-like particle carries a detectable marker gene to detect cell infection. In a preferred embodiment, the marker gene is a fluorescent marker gene, such as GFP, and the infection is detected by means of fluorescence measurement, for instance by flow cytometry analysis of cells contacted with said infectious particles.

A cell suitable to be used in the method of identification of molecules interfering with HCV cell entry may be selected from the group consisting of a hepatocyte cell line and a primary human hepatocyte, as described above.

Such molecules capable of interfering with HCV entry in cells may constitute new antiviral drugs.

The infectious particles of the invention are further useful for diagnosis of HCV infection and follow-up of HCV infection, for instance to assess efficacy of a therapy in a patient.

The invention thus concerns the use of an infectious HCV-like particle for the *in vitro* detection of antibodies directed against HCV in a biological sample from a subject susceptible to be infected with HCV. Said biological sample may be a biological fluid, such as blood or serum, or a tissue biopsy, for instance a tissue sample from liver biopsy. In a specific embodiment, said antibodies are directed against E1 and/or E2 HCV glycoproteins.

Accordingly, the invention provides a method of *in vitro* diagnosis of a HCV infection in a patient comprising detecting immune complexes formed by interaction of anti-HCV antibodies likely to be present in a biological sample of the patient, with HCV-like particle of the invention. Said method may in particular comprise the steps consisting of:
- contacting a biological sample with an infectious HCV-like particle of the invention under conditions sufficient to allow formation of complexes by binding of said infectious particle to antibodies directed against HCV present in the biological sample;
- detecting said complexes, which presence is indicative of a HCV infection.

The presence of antibodies reactive with HCV-like particles can be detected using standard electrophoretic and immunodiagnostic techniques, including immunoassays such as competition, direct reaction, or sandwich type assays. Such assays include, but are not limited to, Western blots; agglutination tests; enzymelabeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the HCV-like particle and the antibody or antibodies reacted therewith.

In another embodiment, said method of *in vitro* diagnosis of a HCV infection in a patient comprises detecting an inhibitory effect of anti-HCV antibodies likely to be present in a biological sample of the patient, on the infection of a permissive cell by a HCV-like particle of the invention. Said method may in particular comprise the steps consisting of:
- contacting a cell permissive for HCV infection with a HCV-like particle and a biological sample;
- comparing cell infectivity measured in presence of said biological sample with cell infectivity measured in absence of said biological sample;
- detecting the inhibition of HCV-like particle infection of a permissive cell as a decrease in cell infectivity measured in presence of said biological sample compared with cell infectivity measured in absence of said biological sample, said inhibition being indicative of a HCV infection.

This embodiment is advantageous in that the method relies on the detection of the specific antibodies that are neutralizing for cell infection, that is those patient's antibodies that are effective against viraemia.

In a further embodiment of this invention, commercial diagnostic kits may be useful to carry out the above diagnosis methods, by detecting the presence or absence of immune complexes formed by HCV particles and antibodies directed against HCV in a biological sample from a subject susceptible to be infected with HCV, or by detecting an inhibition of HCV-like particle infection of a permissive cell by anti-HCV neutralizing antibodies likely to be present in a biological sample of the patient. Such kits may comprise at least a HCV-like particle of the present invention. Where the method involves detection of immune complexes, the kits may further comprise appropriate means of detection of said immune complexes. Preferably the kit of the invention further comprises directions, and protocols, depending upon the method selected, *e.g.*, "competitive", "sandwich", and the like. The kits may also contain peripheral reagents such as buffers, stabilizers, etc..

In another aspect of the invention, the infectious HCV-like particles may be used for vaccination purposes.

According to an embodiment, the invention thus proposes a method of vaccination, notably against HCV infection, that comprises administration of a HCV-like particle to a subject in need thereof. The invention also relates to a vaccine composition comprising a HCV-like particle and a pharmaceutically acceptable carrier. The invention further provides a immunogenic composition comprising in a pharmaceutical acceptable carrier, a HCV-like particle disclosed herein.

The vaccine and immunogenic compositions of the invention may be drawn to confer immunity, or elicit an immune response against HCV.

However, where the HCV-like particles of the invention further carry an additional gene encoding another antigen, different from HCV antigens, the invention provides a recombinant viral vaccine useful to raise an immune response against said antigen. Actually, the use of pseudo-particles described herein makes it possible to improve the elicited immune response through combining several presentation and processing pathways of an antigen. For instance, a vaccine composition of the invention, when administered, results in the HCV-like particles infecting cells of the host. The transgene encoding the antigen is then integrated in the cellular genome, and subsequently expressed by the cell, such that there is both a cellular and a humoral immune response elicited by the vaccine composition.

Advantageously, the HCV-like particles may further carry a transgene encoding an immune modulator, which allows for enhancement of the raised immune reaction.

The vaccination or immunogenic composition of the present invention may additionally contain an adjuvant. A number of adjuvants are known to those skilled in the art. Examples of suitable adjuvants include, for example, include aluminum hydroxide; Saponin; detergents such as Tween 80; animal, mineral or vegetable oils, Corynebacterium or Propionibacterium -derived adjuvants; Mycobacterium bovis (Bacillus Calmette and Guerinn, or BCG); cytokines; acrylic acid polymers such as carbomer; EMA; or combinations thereof.

The route of administration is any conventional route used in the vaccine field. As general guidance, a vaccine composition of the invention is administered via a mucosal surface, *e.g.*, an ocular, intranasal, pulmonary, oral, intestinal, rectal, vaginal, and urinary tract surface; or via a parenteral route, *e.g.*, by an intravenous, subcutaneous, intraperitoneal, intradermal, intraepidermal, or intramuscular route. The choice of administration route depends on the formulation that is selected.

In still another embodiment the particles of the invention may be used as vectors for gene transfer and/or gene therapy. Gene therapy is defined as the introduction of genetic material into a cell in order to either change its phenotype or genotype. Owing to their tropism for hepatic cells, either primary or cancerous, the HCV-like particles described herein comprise an efficient gene delivery system specific for hepatocytes. Furthermore, such a delivery system is amenable to scale up for reproducibly producing large titers of infectious, replication-defective HCV-like particles.

Accordingly, the invention relates to a method for *in vivo* or *in vitro* transferring a transgene of interest in a cell, which method comprises infecting a cell with a HCV-like particle of the invention, wherein the particle carries a transgene of interest.

The invention further relates to the use of a HCV-like particle of the invention, that carries a transgene of interest, for the preparation of a medicament for the prevention or treatment of a disease in a patient, wherein the HCV-like particle allows the transfer of the transgene of interest into a cell of the patient, and encodes a product that has a prophylactic or therapeutic effect against the disease.

Preferably, the targeted cell is a hepatic cell.

The invention will be further understood in view of the following examples and the annexed figures.

### LEGEND TO THE FIGURES :

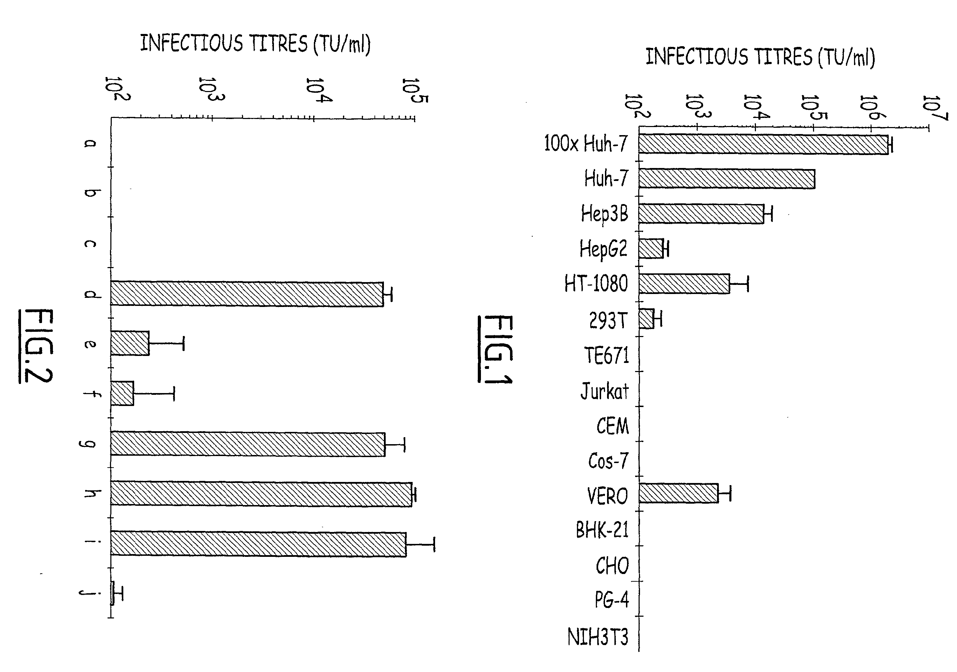

Figure 1 depicts the results of infectivity experiments performed with HCV pseudo-particles of 1a genotype and different target cell types. Results are displayed as transducing units (TU) per ml of supernatant (mean ± standard deviations of up to six experiments) for HCVpp. The infectivity on Huh-7 cells of HCVpp concentrated 100 times by ultra-centrifugation is shown (100x).

Figure 2 represents the infectious titres determined on Huh-7 target cells with HCV pseudo-particles generated without E1 E2 (lane a), without retroviral core proteins (lane b), with MLV-G2A assembly-defective core proteins (lane c), with HIV-1 core proteins (lane d), with E1 or E2 alone (lane e or f, respectively), with E1+E2 expressed in *trans* from two independent vectors (lane g), with HCV-1a E1E2 expressed from the same vector in *cis* (lane h), or with HCV-1b E1 E2 (lane i). HCVpp were treated with 25 µM AZT (3'-azido-3'-deoxythymidine; Sigma-Aldrich, France) before and during infection of target cells (lane j). Results are expressed as TU/ml and are displayed as mean ± standard deviations of up to four experiments.

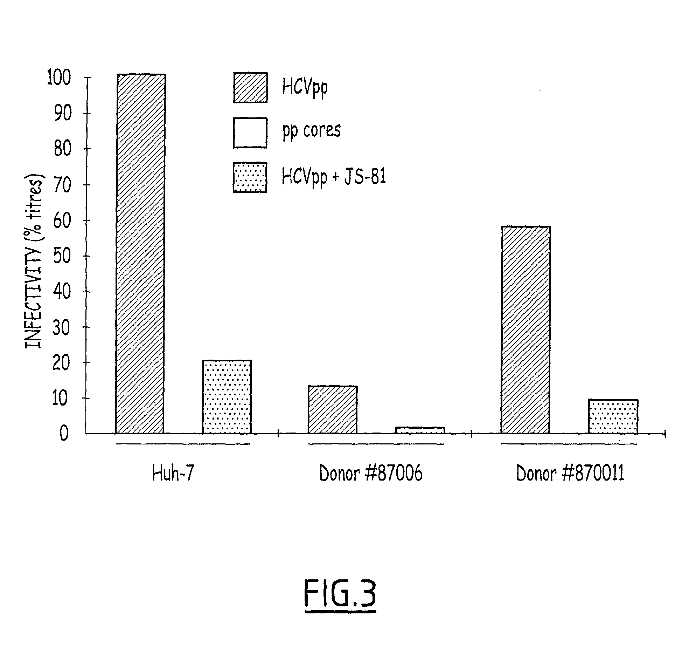

Figure 3 shows the results of infection on human primary hepatocytes derived from two donors with of HCVpp of genotype 1a. Infectivity is expressed as percentage of infectivity determined on Huh-7 cells.

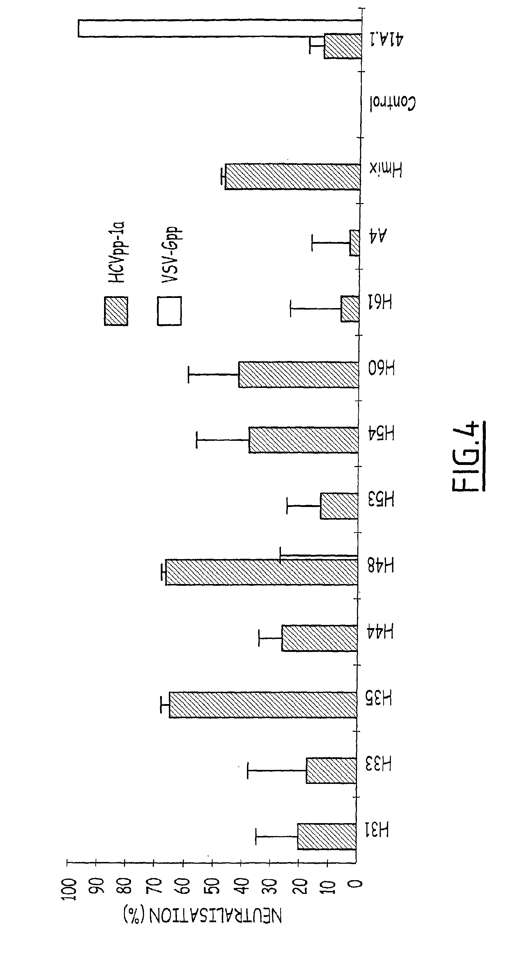

Figure 4 displays neutralisation of HCV pseudo-particles with monoclonal antibodies against E1 (A4) or E2 (H31, H33, H35, H44, H48, H53, H54, H60 and H61) glycoproteins of genotype 1a, with pooled antibodies (Hmix), with no antibodies or using pseudo-particles generated with VSV-G (control). Neutralization of the control was achieved with the VSV-G neutralising 41A.1 monoclonal antibody. Results are expressed as the percentages of inhibition of the average infectious titres ± standard deviations relative to incubation in the absence of antibodies.

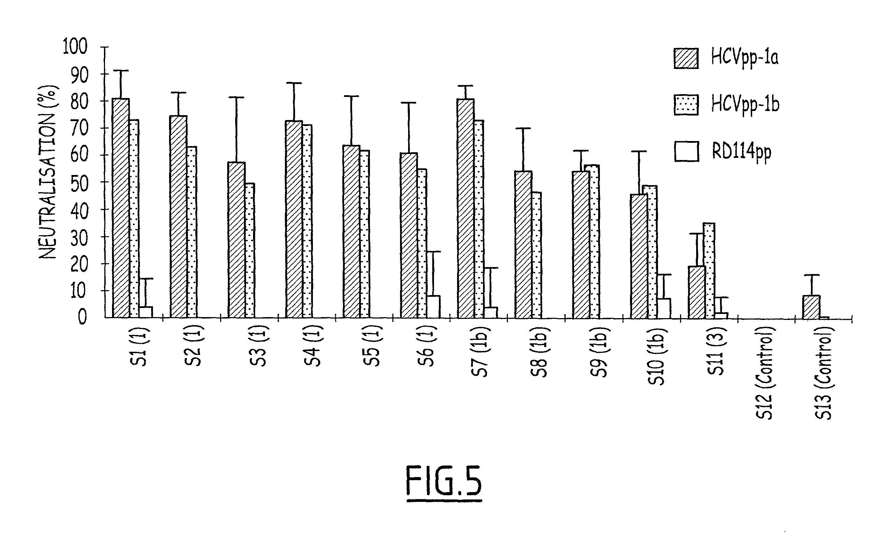

Figure 5 represents neutralisation of HCVpp with HCV patient sera. The genotype of HCV diagnosed in these patients is indicated in brackets. Results are expressed as percentages of inhibition of the average infectious titres ± standard deviations relative to incubation with control sera from healthy individuals.

Figures 6A and 6B show HCV E1E2 and retroviral expression constructs. (A) : A cDNA derived from the HCV polyprotein gene was used to express the E1E2 glycoproteins and the carboxy-terminus of the C protein, which provides the signal peptide for E1 (SP E1). The position of stop codons (star) inserted in the expression constructs to terminate translation of the proteins is shown. The transmembrane domain (TMD) of E1 provides the signal peptide (SP E2) for the E2 glycoprotein. (B) : The expression constructs encoding the different components required to assemble infectious pseudo-particles are shown. The filled boxes represent the viral genes and the marker gene (GFP) transferred to the infected cells. The open boxes show the cis-acting sequences. LTR, long terminal repeat ; CMV, human cytomegalovirus immediate-early promoter; PBS, primer binding site ; ψ, packaging sequence ; PPT, poly-purine track ; polyA, polyadenylation site ; SD, splice donor site ; SA, splice acceptor site. Vector particles were produced by cotransfection of plasmids harboring the packaging functions, the transfer vector and the viral glycoproteins into 293 T cells. The viral glycoproteins were the HCV E1 and E2 glycoproteins, expressed individually or as a CE1E2, the VSV-G or the RD114 glycoproteins. The supernatants of transfected cells were collected during transient expression, concentrated by ultracentrifugation, and used for target cell transduction.

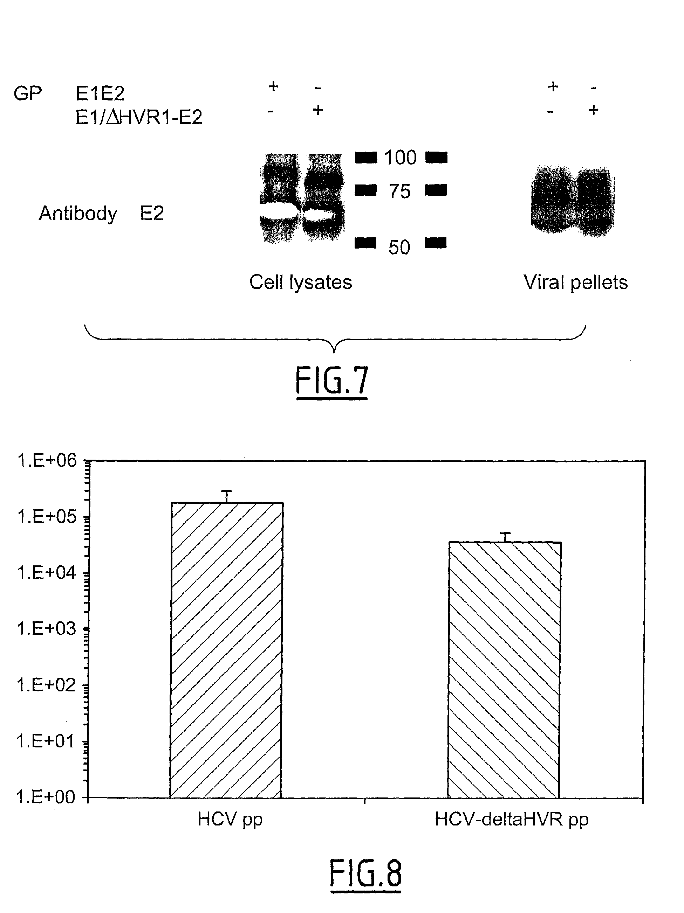

Figure 7 shows incorporation of ΔHVR1 HCV pseudo-particles. Immunoblots of lysates of 293 T transfected cells and of pseudo-particles pelleted through 20 % sucrose-cushions are shown. The positions of the molecular weight markers are shown (kDa).

Figure 8 represents the infectivity of ΔHVR1 HCV pseudo-particles. Supernatants from producer cells were diluted and used to infect Huh-7 target cells before assessing transduction efficiency three days post-infection by FACS analysis.

### EXAMPLES:

### EXAMPLE 1 : Generation of HCV pseudo-particles (HCVpp)

HCV pseudo-particles (HCVpp) were generated by assembling full-length, unmodified E1 and E2 glycoproteins onto retroviral core proteins derived from murine leukemia virus (MLV). To investigate further whether functional HCVpp could also be produced with E1 and E2 expressed in *trans* or with only one of the two glycoproteins, expression vectors that encoded individually either E1 or E2 glycoproteins were designed.

### Construction of expression vectors encoding the viral components, i.e., E1, E2, or E1E2 glycoproteins and viral core proteins

Plasmids expressing wild type E1E2 polyproteins were constructed by standard methods (Sambrook et *al.,* 1989).

Briefly, the phCMV-7a expression vector encoding the E1 and E2 glycoproteins from a 1 a type HCV was generated by inserting the blunted *Cla*I and *Stu*l restriction fragment encoding the last 60 residues of HCV core (C) and all of E1 and E2 proteins from the pTM1p5E1E2(745) vector (Op De Beeck *et al.,* 2000) into the *Bam*Hl digested and Klenow blunted vector phCMV-G (Nègre et *al.,* 2000).

The phCMV-E1 expression vector, expressing only HCV E1 glycoprotein, was derived from phCMV-7a by adding a stop codon to the C-terminus of E1 with primers 5'-actggacgacgcaaagctgc (SEQ ID N°2) and 5'-cgcggatcctacgcgtcgacgccggcaaa (SEQ ID N°3). The resulting PCR fragment was digested with *Bam*Hl and ligated into *Bam*Hl-digested phCMV-7a.

phCMV-E2, expressing only HCV E2 glycoprotein, was obtained by fusing the N-terminus of E2 to the C-terminus of the HCV core using two PCR fragments generated with the two primer pairs 5'-tgcccgcttcagccgaaacccacgtcaccggggga (SEQ ID N°4) + 5'-gccagaagtcagatgctcaagg (SEQ ID N°5) and 5'-tactctgagtccaaaccg (SEQ ID N°6) + 5'-gtgacgtgggtttcggctgaagcgggcacagtcag (SEQ ID N°7). The two PCR fragments were then fused in a second round PCR. The resulting DNA fragment was digested with *Bam*Hl and ligated into *Bam*Hl cut phCMV-7a.

The sequence of the phCMV-ΔC E1 vector is shown in SEQ ID No 8, whereas the aminoacid sequence of E1 protein is shown in SEQ ID No 9. The sequence of the phCMV-ΔC E1 E2 vector is shown in SEQ ID No 10, whereas the aminoacid sequence if the E1E2 polyprotein is shown in SEQ ID No 11. The sequence of the phCMV-ΔC E2 vector is shown in SEQ ID No 12, whereas the aminoacid sequence of E2 protein is shown in SEQ ID No 13.

Expression vectors for E1E2 glycoproteins of 1b genotype were constructed by similar strategies.

HCV E1E2 were therefore expressed from a polyprotein containing the carboxy-terminus of the core (ΔC) protein, which served as signal peptide for E1, E2 or E1 and E2 glycoproteins.

### Generation of HCV pseudo-particles

Retroviruses were chosen as platforms for assembly of HCVpp because their cores can incorporate a variety of different cellular and viral glycoproteins and because they can easily package and integrate genetic markers into host cell DNA.

HCVpp were produced by transfecting 293T human embryo kidney cells (ATCC CRL-1573) with three expression vectors encoding a ΔCE1E2, ΔCE1 or ΔCE2 polyprotein, the MLV core proteins and a packaging-competent MLV-derived genome harbouring the GFP (green fluorescent protein) marker gene. This construct contains the GFP marker gene, whose expression is driven by the CMV (cytomegalovirus) immediate early promoter. Both CMV and GFP nucleic acid sequences where inserted in a retroviral vector derived from MLV in which the gag, pol and env viral gene where removed and in which the retroviral cis-acting elements that control vector genome packaging, reverse transcription and integration were retained.

Control pseudo-particles were generated with the VSV-G glycoprotein (Nègre *et al.,* 2000), the RD114 virus envelope glycoprotein (Sandrin *et al.,* 2002), and/or with assembly-defective MLV core proteins (MLV-G2A) (Swanstrom et *al.*, 1997).

Briefly, expression constructs were transfected into 2.5x10⁶ 293T cells seeded the day before in 10 cm plates using a calcium-phosphate transfection protocol (Clontech, France) according to the manufacturer's recommendations. The medium (8 ml/plate) was replaced 16 hrs after transfection. Supernatants containing the pseudo-particles were harvested 24 hrs later, filtered through 0.45µm-pore-sized membranes and processed as described before (Nègre *et al.,* 2000).

### Immunoblot analysis of structural components of the pseudo-particles

Lysates of transfected cells and of pseudo-particles pelleted through 20% sucrose-cushions were immunobloted. Expression of E1 and E2 glycoproteins from HCV-1a genotype and of MLV core proteins was revealed in reducing and denaturing conditions with monoclonal antibodies against E1 (A4) (Dubuisson et *al.,* 1994) and E2 (H52) (Flint *et al.,* 1999) or with an anti-capsid (MLV CA) antiserum (ViroMed Biosafety Laboratories, USA), as described previously (Sandrin *et al.,* 2002). VSV-G expressed in control pseudo-particles was detected with the monoclonal antibody P5D4 (Sigma-Aldrich, France).

Analysis of immunoblots of transfected cells showed that the structural components of the pseudo-particles were readily detected at the expected molecular weights; *i.e.* 30 kDa for E1, 60 kDa for E2, 60 kDa for VSV-G and 70 kDa for the RD114 glycoprotein. MLV core proteins were detected as a Gag protein precusor of 65 kDa that was partially processed by the MLV protease into mature core components.

The E1 and E2 glycoproteins were readily detected in the pellets of purified virions generated with the wild-type MLV core particles but not with the viral assembly-deficient MLV-G2A mutant. E2 present within the viral pellets migrated slightly slower than the cell-associated forms, due to modifications of the associated glycans by Golgi enzymes.

The presence of VSV-G in viral pellets generated with MLV-G2A assembly-defective core proteins is due to empty vesicles formed by VSV-G itself (Nègre *et al.,* 2000).

Comparison of the relative levels of VSV-G or HCV glycoproteins in producer cell lysates versus viral pellets suggests efficient incorporation of E1 and E2 into viral particles. Likewise, could E1 and E2 glycoproteins efficiently assemble on retroviral core proteins derived from HIV-1, raising the possibility of pathogenic interactions between the two parental viruses, *in vivo,* as co-infection of patients with HCV and HIV is prevalent (Dieterich, 2002).

Altogether, these results indicate that transient over-expression of E1 and E2 in 293T cells leads to specific and efficient incorporation of HCV glycoproteins into pseudo-particles generated with retroviral cores. Since HCV envelope glycoproteins have been shown to be retained in the ER (Op De Beeck et al. 2001), this implies that HCVpp form by budding into the ER lumen or, alternatively, that saturation/leakiness of the ER retention allows a fraction of E1E2 to reach the cell surface where MLV budding normally occurs (Swanstrom and Wills, 1997).

Individual expression or co-expression in *trans* of E1 and E2, from distinct expression units, led to normal levels of synthesis, as compared to expression of both glycoproteins in *cis,* from a single E1 E2 polyprotein. Moreover, HCVpp were found to incorporate similar levels of E2 glycoprotein, whether it was expressed alone, or co-expressed in *cis* or in *trans* with E1. Finally, incorporation of E1 expressed alone or co-expressed in *trans,* with E2, occurred but at reduced levels, consistent with the chaperone activity of E2 (Op De Beeck et *al.,* 2001). Formation of HCVpp carrying E1 or E2 only will allow investigation of their respective roles in HCV cell entry and infectivity.

### EXAMPLE 2 : HCVpp cell line infectivity

Infectivity of HCVpp was assessed on a panel of target cell lines : Huh-7 human hepatocellular carcinoma (Nakabayashi et *al.*, 1982), Hep3B human hepatocellular carcinoma (ATCC HB-8064), HepG2 human hepatocellular carcinoma (HB-8065), HT-1080 human fibrosarcoma (CCL-*121*), 293T, TE671 human rhabdomyosarcoma (ATCC CRL-8805), Jurkat human T cell leukemia (TIB-152), CEM human lymphoblastic leukemia (CCL-119), COS-7 African green monkey fibroblasts kidney (CRL-1651 ), VERO African green monkey kidney (CCL-81 ), PG-4 feline astrocyte (CRL-2032), BHK-21 golden hamster kidney (CCL-10), CHO Chinese hamster ovary (ATCC CCL-61), and NIH3T3 mouse fibroblasts. Target cells were grown as recommended by the ATCC.

Target cells (seeded the day before 8x10⁴ cells/well in 12-well plates) were incubated for 3 hrs with dilutions of supernatants from producer cells containing the HCVpp, then washed and cultured until expression of the GFP marker gene harboured by the virions was assessed by FACS analysis 72 hrs later (Sandrin *et al.,* 2002). Since the HCVpp were generated with replication-defective viral components, this procedure allowed evaluation of the specific infectivity of the pseudo-particles after a one-round infection process.

Infectious titres of up to 1.1x10⁵ TU/ml were detected for the HCVpp on Huh-7 human hepato-carcinoma cells (Figure 1). Upon concentration of the producer cell supernatants by ultra-centrifugation (Sandrin *et al.,* 2002), infectious titres of 2x10⁶ TU/ml, on average, could be readily obtained. The other target cell types used in the infection assays displayed weaker (Hep3B, HepG2, HT1080, 293T, Vero) or undetectable (TE671, Jurkat, CEM, Cos-7, BHK-21, CHO, PG-4, NIH3T3) levels of infection with the HCVpp. The infectivity of control pseudo-particles generated with VSV-G ranged from 5x10⁶ to 2x10⁷ TU/ml depending on the target cell type indicating that all these cells were readily infected with the control pseudo-particles. This suggested that all the molecules necessary for HCV entry were specifically expressed in the former cell types only.

Infectious HCVpp could be generated at comparable efficiencies with E1E2 glycoproteins derived from HCV genotypes 1a and 1b (lanes h and i; Figure 2) and/or with retroviral core proteins derived from either HIV-1 or MLV (lanes d and h; Figure 2).

Incubation of HCVpp and target cells with AZT, a reverse-transcriptase inhibitor that prevents conversion of the retroviral RNA genome of the HCVpp as integration-competent proviral DNA, inhibited transduction (lane j; Figure 2).

Moreover, long-term expression of GFP could be demonstrated after serial passaging of the infected cells for more than one month.

These results indicated that infection of the target cells led to integration in host cell DNA and stable expression of the GFP marker gene transduced by the HCVpp. Additionally, no infection could be obtained with viral particles lacking both E1 and E2 glycoproteins, or lacking core proteins, or, alternatively, when using the MLV-G2A assembly-defective core proteins (lanes a-c; Figure 2).

Altogether, these results demonstrated that HCVpp harbouring the E1 and E2 glycoproteins and retroviral core proteins were infectious, leading to retroviralmediated integration of their vector genome.

Finally, despite reduced levels of E1 incorporation, HCVpp generated with E1 and E2 glycoproteins expressed in *trans*, from distinct vectors, were nearly as infectious as those generated with the E1E2 polyprotein construct (lanes g and h; Figure 2). However, HCVpp assembled with either E1 or E2 glycoproteins only were 500-fold less infectious (lanes e-f; Figure 2), demonstrating that both glycoproteins need to be co-incorporated on the HCVpp to allow efficient virus entry and infection.

### EXAMPLE 3 : HCVpp primary hepatocyte infectivity

Hepatocytes represent the principal site of HCV replication *in vivo,* yet *ex vivo* studies have suggested that HCV may also infect lymphoid cells (Lindenbach and Rice, 2001). To address whether either cell types could be infected, human adult hepatocytes (Figure 3) and peripheral blood mononuclear cells (PBMCs) have been transduced. Pseudo-particles were generated with core proteins derived from HIV-1 rather than from MLV, which do not permit transduction of non-proliferating target cells (Nègre *et al.,* 2000).

Cryopreserved primary hepatocytes, isolated from human adult biopsy samples checked for absence of HBV, HCV and HIV, were purchased from Biopredic International (Rennes, France) and cultured on collagen I coated plates according to recommendations of the supplier. Transduction of hepatocytes was determined by counting GFP-positive cells under UV microscope. Specificity of infection was demonstrated by absence of transduction when target cells were infected with pseudo-particles lacking glycoproteins (pp cores) or by the reduced levels of transduction when target cells were pre-incubated with JS-81 anti-CD81 antibodies (30 µg/ml) before infection. Infection of human PBMCs, isolated from healthy donors, was conducted as described previously (Sandrin *et al.,* 2002).

Relative to infection of Huh-7 cells, the HCVpp could readily infect the primary hepatocytes derived from different donors (Figure 3), yet transduction efficiencies varied with quality and cell culture viability of the individual biopsies. In contrast, poor or undetectable infection of PBMCs was found with the HCVpp although control pseudo-particles generated with VSV-G could readily infect these primary cells.

Altogether these data indicate that HCVpp closely mimic the early events of infection by wild-type HCV and preferentially infect hepatocytes and hepato-carcinoma cells.

### EXAMPLE 4 : HCVpp is a valid model of early steps of HCV infection

### Infectivity of HCVpp is mediated by E1 and E2 glycoproteins

It was further determined whether infectivity of the HCVpp is specifically mediated by E1 and E2 and their interaction with cell surface receptors. A panel of monoclonal antibodies previously shown to specifically react against HCV-1a glycoproteins (Dubuisson et *al.,* 1994; Flint et *al.,* 1999; Patel et *al.,* 2000) was used.

HCVpp generated with HCV-1 a E1E2 and with MLV core proteins were pre-incubated before infection of Huh-7 cells with saturating concentrations (20 µg/ml) of monoclonal antibodies against E1 (A4) or E2 (H31, H33, H35, H44, H48, H53, H54, H60 and H61) glycoproteins of genotype 1a, or with pooled antibodies (Hmix). Control experiments were performed using no antibodies or using pseudo-particles generated with VSV-G (VSV-Gpp).

Some of these monoclonal antibodies, like H35 and H48, for example, could neutralise the HCVpp of genotype 1a and reduce their infectivity by up to 70% (Figure 4). In contrast, none of these genotype 1a-specific antibodies could neutralise the HCVpp of genotype 1b or the control pseudo-particles generated with VSV-G (Figure 4). Neutralisation of the infectivity of these control pseudo-particles was achieved by using the VSV-G neutralising 41A.1 monoclonal antibody.

These data therefore demonstrate that infectivity of the pseudo-particles is specifically due to the incorporation of E1 and E2 glycoproteins, indicating that these HCVpp represent a valid model to investigate the early steps of HCV infection, *i.e.* receptor binding, membrane fusion and envelope uncoating.

### HCV infection is neutralised by serum from HCV-infected patients

The capacity of sera derived from chronically HCV-infected patients to neutralise infectivity of HCVpp was further assessed.

HCVpp of genotypes 1a or 1b were pre-incubated for 30 min at room temperature with sera from chronically HCV-infected patients diluted 1/50 before infection of Huh-7 target cells. Control experiments were performed using pseudo-particles generated with RD114 glycoproteins (RD114pp), rather than with VSV-G that exhibits sensitivity to human complement (Sandrin et *al.,* 2002). Efficient neutralisation of the control pseudo-particles was demonstrated using a hyper-immune goat serum raised against the RD114 SU glycoprotein (ViroMed Biosafety Laboratories, USA).

No or only weak neutralisation of control pseudo-particles could be detected using the sera of the HCV-infected patients. In contrast, most if not all of these sera could neutralise the infectivity of the HCVpp, in contrast to sera derived from healthy donors. Variable levels of neutralisation were detected depending on the donor and ranged from 20% to up to 90% of inhibition for HCVpp of both genotypes 1a and 1b. Sera derived from patients infected with HCV of the 1b genotype could neutralise the HCVpp generated with E1E2 of genotype 1a or 1b with similar efficiencies (Figure 5).

### EXAMPLE 5 : Cell entry receptor usage by HCV pseudo-particles

Both the LDL receptor (LDLr) and CD81, a member of the tetraspanin family of receptors, have been proposed as putative HCV receptors (Pileri et *al.*, 1998; Agnello et *al.*, 1999). However, recent studies have questioned the role of these molecules as cell entry receptors despite their unequivocal capacity to bind HCV particles and/or glycoproteins (*2023*). Thus, the contribution of either cell surface molecules in the early stages of HCV cell entry was investigated by performing receptor-competition assays.

### LDLr

LDLr binds apoliprotein B within LDL and apoliproteins B and E (ApoB and ApoE) within VLDL complexes; both complexes have been found associated with HCV particles in plasma of infected patients (Andre et *al.*, 2002).

LDL receptor competition assays were performed using purified LDLs (10 µg/ml, 100 µg/ml; Sigma-Aldrich, France) or with the monoclonal antibodies 5E11 (10 µg/ml, 50 µg/ml), 4G3 (anti-ApoB; 10 µg/ml, 50 µg/ml) or 1B7 (anti-ApoE; 1 µg/ml , 10 µg/ml, 50 µg/ml, 100 µg/ml) (Ottawa Heart Institute Research corporation, Ottawa, Ontario, Canada), or mixed 5E11, 4G3 and 1B7 antibodies (with individual concentrations 10 µg/ml, 50 µg/ml), pre-incubated with the pseudo-particles prior to infection.

Purified LDLs were not found to out-compete infection of HCVpp on Huh-7 LDLr-positive cells, even when concentrations higher than 100 µg/ml were used. When monoclonal antibodies targeted to the receptor binding sites of ApoB and ApoE were pre-incubated with the HCVpp, only the anti-ApoE antibodies could inhibit, albeit weakly, the infectivity of the HCVpp. This is consistent with our observation that HCVpp could not or hardly infect LDLr-positive cell lines such as HepG2, Jurkat, CEM and TE671 (Figure 1). Thus, although these results do not exclude a role of LDLr in HCV entry, possibly *via* association of LDLs with HCV particles (Andre et *al.*, 2002), they suggest that LDLr is not the major receptor of HCVpp entry.

### CD81

Recombinant GST-fusion polypeptides encompassing the large extra-cellular loop (LEL) of human CD81 which has been shown to bind HCV E2 (Flint *et al.,* 1999, (Pileri et *al.*, 1998) were then used to investigate the role of hCD81 in HCVpp cell entry.

hCD81-LEL GST-fusion polypeptides (4 µg/ml, 8 µg/ml, 16 µg/ml) were pre-incubated with the pseudo-particles before infection or JS-81 anti-CD81 antibodies (4 µg/ml, 10 µg/ml, 30 µg/ml; Pharmingen, France) were pre-incubated with the target cells prior to infection. Percentages of inhibition of the infectious titres obtained on Huh-7 cells relative to titres obtained in the absence of inhibitors were calculated. Control experiments were performed using pseudo-particles generated with VSV-G (VSV-Gpp). Comparative experiments were further conducted with NIH3T3, NIH3T3-hCD81 and Huh-7 cells infected with HCVpp and with control pseudo-particles generated with VSV-G.

The GST-fusion hCD81-LEL polypeptides pre-incubated with the HCVpp could specifically precipitate E1E2 complexes, confirming their capacity to bind the E2 glycoprotein (Flint *et al.,* 1999; Pileri et *al.,* 1998), and were found to neutralise the infectivity of the HCVpp on Huh-7 cells, yet with a relatively poor efficiency. Inhibition ranged from 38% to 54% inhibition of infectivity and appeared to be dose-dependent.

Consistently, pre-incubation of Huh-7 cells with JS-81 monoclonal antibody, that blocks binding of recombinant E2 to hCD81 (Flint *et al.,* 1999), was found to inhibit infectivity of HCVpp in both Huh-7 target cells and human primary hepatocytes. At high antibody concentrations (30 µg/ml), over 90% inhibition of infection could be obtained for HCVpp based on genotypes 1a as well as 1b. However, several target cells non-permissive to infection by HCVpp, like TE671, Jurkat, and CEM cells (Figure 1) were found to express high densities of hCD81, indicating the lack of correlation between CD81 expression and infectivity.

Moreover, expression of hCD81 in non-permissive NIH3T3 mouse fibroblasts did not allow infection with the HCVpp (Figure 4C).

Altogether these results demonstrate that although hCD81 binds HCV E2 and might help cell surface attachment of HCV, it is not sufficient by itself to allow infection with HCVpp.

### EXAMPLE 6 : Deletion of hypervariable region I

HCV infection is not cleared by over 80% of patients and chronicity is associated with various forms of liver disease. Chronic infection is thought to be caused by the high mutation rate of HCV, which helps the virus to escape the host immune response. One of several mutation hotspots within the HCV genome has been localised to the N-terminus of the E2 glycoprotein. This so-called hypervariable region I is a major antigen and is thought to play an important role in allowing HCV to escape the host's immune response. The antigenic variation of the HVR1 also inhibits the development of a protective immune response against re-infection with heterologous HCV strains/genotypes. Finally does the antigenic dominance and extreme variability of the HVR1 hinder the development of vaccines against HCV. With the long-term aim to develop effective therapeutics which target more conserved parts of the HCV glycoproteins the inventors investigated whether the HVR1 is dispensable for infection.

For that purpose expression constructs encoding the polyprotein for wildtype E1 E2 or a mutant version in which the HVR1 = the first 27 residues of E2 had been deleted from the polyprotein were generated. HCV pseudo-particles (HCVpp) were produced with either wildtype or ΔHVR1 ΔCE1E2 polyprotein. Analysis of immunoblots of transfected cells showed that both wildtype and mutant E2 proteins were expressed to similar levels. Viral particles were harvested from the supernatant of transfected cells and purified by ultra-centrifugation through high-density sucrose cushions. ΔHVR1 and wildtype E2 were incoporated into particles to a similar level (Fig. 7). The infectivity of the wildtype and mutant HCVpp were tested as before on Huh-7 target cells. Infectious titres of up to 2.6x10⁵ TU/ml were detected for wildtype HCVpp (Fig. 8). ΔHVR1 HCVpp were about 5 to 10 fold less infectious, still a maximal titer of 5.1x10⁵ TU/ml was observed suggesting that the HVR1 aids but is not required for the infection process.

In conclusion, the invention provides a tool that allows precise investigation of viral assembly of E1E2 glycoproteins (processing, maturation) and their role in cell entry of HCV. No structural modifications of the E1E2 glycoproteins were required for their correct assembly on retroviral and lentiviral cores and to generate high titre infectious HCVpp with functional E1E2 glycoproteins. The insertion of a marker gene into the HCVpp allowed precise and rapid determination of the infectivity of these pseudo-particles by flow-cytometry.

The development of these functional, infectious HCV pseudo-particles make it now possible to investigate early events of HCV infection, such as the identification of novel HCV receptor(s) or co-receptor(s), to carry out diagnostic assays for the detection of neutralising antibodies in seroconverted patients, and to develop efficient inhibitors to HCV infection.

### REFERENCES

Agnello V. et *al., Proc Natl Acad Sci USA* **96,** 12766-71. (1999).

Andre P. et *al., J Virol* **76**, 6919-28. (2002).

Ausubel F.M. et *al.* (eds.), Current Protocols in Molecular Biology, John Wiley & Sons, Inc. (1994).

Baumert T. F., Ito S., Wong D. T., Liang T. J., *J Virol* **72**, 3827-36. (1998).

Blight K. J., Kolykhalov A. A., Rice C. M., *Science* **290,** 1972-4. (2000).

Buonocore L., Blight K. J., Rice C. M., Rose J. K., *J Virol* **76**, 6865-72. (2002).

Dieterich (2002) *J Infect Dis* **185,** S128-37.

Dubuisson J. et *al., J Virol* **68,** 6147-60. (1994).

Felgner et *al. Science* **337,** 387-388. (1989).

Flint M. et *al., J Virol* **73**, 6235-44. (1999).

Guguen-Guillouzo and Guillouzo. Methods for preparation of adult and fetal hepatocytes. p 1-12. In A. Guillouzo and C. Guguen-Guillouzo (ed.), Isolated and cultured hepatocytes. Les Editions INSERM Paris. John Libbey and Co, Ltd., London, United Kingdom (1986).

Lavanchy D. et *al., J. Viral Hepat* **6**, 35-47 (1999).

Lindenbach B. D., Rice C. M., *Flaviviridae: The Viruses and Their Replication.* Knipe D. M., Howley P. M., Eds., Fields Virology, 4th ed. (Lippincott Williams & Wilkins, Philadelphia, Pa, 2001).

Lohmann V. et *al., Science* **285**, 110-3. (1999).

Matsuura Y. et *al., Virology* **286,** 263-75. (2001).

Nakabayashi H. et *al., Cancer Res* **42**, 3858-63. (1982).

Nègre D. et *al., Gene Ther***7**, 1613-1623 (2000).

Op De Beeck A. et *al., J Biol Chem* ***275,*** 31428-37. (2000).

Op De Beeck A., L. Cocquerel, J. Dubuisson, *J Gen Virol* **82,** 2589-95. (2001).

Owsianka A. et *al., J Gen Virol* **82**, 1877-83. (2001).

Patel A. H. et *al., J Gen Virol* **81,** 2873-83. (2000).

Pietschmann T. et *al., J Virol* **76,** 4008-21. (2002).

Pileri P. et *al., Science* **282,** 938-41. (1998).

Rose N. F. et *al., Cell* **106,** 539-49. (2001).

Sambrook, J., Fritsch, E. F., and Maniatis, T. *Molecular Cloning: A Laboratory Manual ,* 2nd Ed. , Cold Spring Harbor Laboratory, Cold Spring Harbor, NY (1989).

Sandrin V. et *al., Blood* **100**, 823-832 (2002).

Swanstrom R., Wills J. W., in *Retroviruses* J. M. Coffin, S. H. Hughes, H. E. Varmus, Eds. (Cold Spring Harbor Laboratory Press, New York, USA, 1997) pp. 263-334.

Wellnitz S. et *al., J Virol* **76***,* 1181-93. (2002).

Wu *et al.,* (1988) J. Biol. Chem. 263:14621-14624.

Wu *et al.,* (1992) J. Biol. Chem. 267:963-967.

## Claims

1. A method for producing hepatitis C virus (HCV)-like particles ex vivo comprising the steps of:
- providing a first nucleic acid sequence comprising a packaging competent retroviral-derived genome;
- providing a second nucleic acid sequence comprising a cDNA encoding core proteins from said retrovirus;
- providing a third nucleic acid sequence comprising a cDNA encoding a polyprotein comprising successively a HCV core protein, and a HCV E1 protein and/or a HCV E2 protein;
- transfecting host cells with said nucleic acid sequences and maintaining the transfected cells in culture for sufficient time to allow expression of the cDNAs to produce structural proteins from hepatitis C virus and retrovirus; and allowing the structural proteins to form virus-like particles.

2. The method according to claim 1, wherein said packaging competent retroviral-derived genome and core proteins are from a retrovirus selected from the group consisting of MLV, ALV, RSV, MPMV, HIV-1, HIV-2, SIV, EIAV, CAEV, or HFV.

3. The method according to claim 1 or 2, wherein said HCV core protein comprises the last 21 amino acids of the carboxy-terminus of HCV core

4. The method according to any of claims 1 to 3, wherein said polyprotein comprises a HCV core protein and a HCV E1 protein.

5. The method according to any of claims 1 to 3, wherein said polyprotein comprises a HCV core protein and a HCV E2 protein.

6. The method according to any of claims 1 to 5, comprising native HCV E1 and/or E2 protein.

7. The method according to any of claims 1 to 3, wherein said polyprotein comprises a HCV core protein, native HCV E1 protein and native HCV E2 protein.

8. The method according to any of preceding claims, wherein said nucleic acid sequence comprising a packaging competent retroviral-derived genome further comprises a transgene.

9. An infectious HCV-like particle susceptible to be obtained by a method according to any of preceding claims, comprising the core proteins from a retrovirus, and a E1 HCV glycoprotein and/or a E2 HCV glycoprotein.

10. The infectious particle according to claim 9, comprising E1 and E2 HCV glycoproteins.

11. The infectious particle according to claim 9, comprising E1 HCV glycoprotein.

12. The infectious particle according to claim 9, comprising E2 HCV glycoprotein.

13. The infectious particle according to any of claims 9 to 12, comprising native E1 and/or E2 HCV glycoprotein.

14. The infectious particle according to any of claims 9 to 13, wherein said retrovirus is selected from the group consisting of MLV, ALV, RSV, MPMV, HIV-1, HIV-2, SIV, EIAV, CAEV, or HFV.

15. The infectious particle according to any of claims 9 to 14, wherein said nucleic acid sequence comprising a packaging competent retroviral-derived genome further comprises a transgene.

16. Use of three nucleic acid sequences for the preparation of a medicament useful as a vaccine against hepatitis C, wherein the nucleic acid sequences are :
- a first nucleic acid sequence comprising a packaging competent retroviral-derived genome;
- a second nucleic acid sequence comprising a cDNA encoding core proteins from said retrovirus;
- a third nucleic acid sequence comprising a cDNA encoding a polyprotein comprising successively a HCV core protein, and a HCV E1 protein and/or a HCV E2 protein ;
and, when transferred into cells of a subject, the nucleic acids sequences allow the production of structural proteins from hepatitis C virus and retrovirus, wherein the structural proteins form virus-like particles that are immunogenic.

17. A method for *ex vivo* identification of a receptor for HCV E1 and/or E2 glycoprotein comprising detection of the binding of an infectious particle according to any of claims 9 to 14, to a cell receptor.

18. A method for *ex vivo* identifying a cell receptor for HCV comprising the step consisting of:
- transfecting a cell which is not permissive for HCV infection with a nucleic acid sequence encoding a protein likely to be a receptor for HCV;
- contacting said transformed cell with a HCV-like particle according to any of claims 9 to 14;
- determining whether said transformed cell has become permissive or not for HCV infection; and
- identifying as a cell receptor for HCV said protein expressed by the transformed cell that has become permissive.

19. A method for *ex vivo* identifying a cell receptor for HCV comprising the step consisting of:
- providing an expression cDNA library obtained from a cell permissive for HCV infection;
- transfecting cells that are not permissive for HCV infection with said expression cDNA library;
- contacting said transformed cells with HCV-like particles according to any of claims 9 to 14;
- identifying and isolating those transformed cells that have become permissive for HCV infection;
- isolating the expression vector transfected in cells that have become permissive; and
- identifying as a receptor for HCV the proteins encoded by the cDNA sequence of said isolated expression vectors.

20. A method of *ex vivo* screening or identification of molecules capable of interfering with HCV entry in cells comprising comparison of the level of cell infection by an infectious particle according to any of claims 9 to 14 in the presence or the absence of a candidate molecule.

21. A method of *in vitro* diagnosis of a HCV infection in a patient, comprising detecting immune complexes formed by interaction of anti-HCV antibodies likely to be present in a biological sample of the patient with HCV-like particle according to any of claims 9 to 14.

22. A method of *in vitro* diagnosis of a HCV infection in a patient, comprising detecting an inhibitory effect of anti-HCV antibodies likely to be present in a biological sample of the patient, on the infection of a permissive cell by HCV-like particles according to any of claims 9 to 14.

23. A diagnostic kit useful for the method of claim 21, comprising a HCV-like particle according to any of claim 9 to 14 and appropriate means of detection of said immune complexes.

24. Vaccine composition comprising a HCV-like particle according to any of claims 9 to 15 and a pharmaceutically acceptable carrier.

25. A method for *in vitro* transferring a transgene of interest in a hepatic cell comprising infecting a cell with a HCV-like particle as described in any of claims 9 to 14, wherein the HCV-like particle carries a transgene of interest.

26. Use of a HCV-like particle according to any of claims 9 to 14, that carries a transgene of interest, for the preparation of a medicament for the prevention or treatment of a disease in a patient, wherein the HCV-like particle allows the transfer of the transgene of interest into a cell of the patient, and encodes a product that has a prophylactic or therapeutic effect against the disease.


SEQUENCE LISTING

<110> INSERM

<120> Infectious HCV-pseudo particles containing functional
      E1,E2 envelope proteins

<130> BET02/0805

<140>
<141>

<160> 13

<170> PatentIn Ver. 2.1

<210> 1
<211> 21
<212> PRT
<213> Hepatitis C virus

<400> 1
Gly Cys Ser Phe Ser Ile Phe Leu Leu Ala Leu Leu Ser Cys Leu Thr
  1               5                  10                  15

Val Pro Ala Ser Ala
             20


<210> 2
<211> 20
<212> DNA
<213> Artificial Sequence

<220>
<223> Description of Artificial Sequence: primer

<400> 2
actggacgac gcaaagctgc                                             20


<210> 3
<211> 29
<212> DNA
<213> Artificial Sequence

<220>
<223> Description of Artificial Sequence: primer

<400> 3
cgcggatcct acgcgtcgac gccggcaaa                                   29


<210> 4
<211> 35
<212> DNA
<213> Artificial Sequence

<220>
<223> Description of Artificial Sequence: primer

<400> 4
tgcccgcttc agccgaaacc cacgtcaccg gggga                                35

<210> 5
<211> 22
<212> DNA
<213> Artificial Sequence

<220>
<223> Description of Artificial Sequence: primer

<400> 5
gccagaagtc agatgctcaa gg                                              22

<210> 6
<211> 18
<212> DNA
<213> Artificial Sequence

<220>
<223> Description of Artificial Sequence: primer

<400> 6
tactctgagt ccaaaccg                                                   18

<210> 7
<211> 35
<212> DNA
<213> Artificial Sequence

<220>
<223> Description of Artificial Sequence: primer

<400> 7
gtgacgtggg tttcggctga agcgggcaca gtcag                                35

<210> 8
<211> 6274
<212> DNA
<213> Artificial Sequence

<220>
<223> Description of Artificial Sequence::
      phCMV-deltaCE1-1a plasmid

<220>
<221> CDS
<222> (2116)..(2880)

<220>
<221> promoter
<222> (1)..(768)
<223> human cytomegalovirus (hCMV) immediate-early promoter

<220>
<221> misc_feature
<222> (769)..(1429)

<223> rabbit beta-globin intron II

<220>
<221> misc_feature
<222> (1425)..(1430)
<223> BamHI 5'junction after ligation of deltaCE1
      fragment in BamHI site of hCMV-G

<220>
<221> misc_feature
<222> (1430)..(2115)
<223> IRES sequence of EMCV

<220>
<221> misc_feature
<222> (2125)..(2304)
<223> deltaC part of the polyprotein

<220>
<221> misc_feature
<222> (2305)..(2880)
<223> Mature E1 protein

<220>
<221> misc_feature
<222> (2883)..(2888)
<223> BamHI 3' junction after ligation of deltaCE1
      fragment in BamHI site of hCMV-G

<400> 8
gcggccgctc tagagagctt ggcccattgc atacgttgta tccatatcat aatatgtaca 60

tttatattgg ctcatgtcca acattaccgc catgttgaca ttgattattg actagttatt 120

aatagtaatc aattacgggg tcattagttc atagcccata tatggagttc cgcgttacat 180

aacttacggt aaatggcccg cctggctgac cgcccaacga cccccgccca ttgacgtcaa 240

taatgacgta tgttcccata gtaacgccaa tagggacttt ccattgacgt caatgggtgg 300

agtatttacg gtaaactgcc cacttggcag tacatcaagt gtatcatatg ccaagtacgc 360

cccctattga cgtcaatgac ggtaaatggc ccgcctggca ttatgcccag tacatgacct 420

tatgggactt tcctacttgg cagtacatct acgtattagt catcgctatt accatggtga 480

tgcggttttg gcagtacatc aatgggcgtg gatagcggtt tgactcacgg ggatttccaa 540

gtctccaccc cattgacgtc aatgggagtt tgttttggca ccaaaatcaa cgggactttc 600

caaaatgtcg taacaactcc gccccattga cgcaaatggg cggtaggcgt gtacggtggg 660

aggtctatat aagcagagct cgtttagtga accgtcagat cgcctggaga cgccatccac 720

gctgttttga cctccataga agacaccggg accgatccag cctccggtcg accgatcctg 780

agaacttcag ggtgagtttg gggacccttg attgttcttt ctttttcgct attgtaaaat 840

tcatgttata tggagggggc aaagttttca gggtgttgtt tagaatggga agatgtccct 900

tgtatcacca tggaccctca tgataatttt gtttctttca ctttctactc tgttgacaac 960

cattgtctcc tcttattttc ttttcatttt ctgtaacttt ttcgttaaac tttagcttgc 1020

atttgtaacg aatttttaaa ttcacttttg tttatttgtc agattgtaag tactttctct 1080

aatcactttt ttttcaaggc aatcagggta tattatattg tacttcagca cagttttaga 1140

gaacaattgt tataattaaa tgataaggta gaatatttct gcatataaat tctggctggc 1200

gtggaaatat tcttattggt agaaacaact acaccctggt catcatcctg cctttctctt 1260

tatggttaca atgatataca ctgtttgaga tgaggataaa atactctgag tccaaaccgg 1320

gcccctctgc taaccatgtt catgccttct tctctttcct acagctcctg ggcaacgtgc 1380

tggttgttgt gctgtctcat cattttggca aagaattcct cgacggatcc gatgaaggac 1440

agttctttcc agacattgtt gaattgatct cgatcccgcg aaattaatac gactcactat 1500

agggagacca caacggtttc cctctagcgg gatcaattcc gcccctctcc ctcccccccc 1560

cctaacgtta ctggccgaag ccgcttggaa taaggccggt gtgcgtttgt ctatatgtta 1620

ttttccacca tattgccgtc ttttggcaat gtgagggccc ggaaacctgg ccctgtcttc 1680
ttgacgagca ttcctagggg tctttcccct ctcgccaaag gaatgcaagg tctgttgaat 1740

gtcgtgaagg aagcagttcc tctggaagct tcttgaagac aaacaacgtc tgtagcgacc 1800

ctttgcaggc agcggaaccc cccacctggc gacaggtgcc tctgcggcca aaagccacgt 1860

gtataagata cacctgcaaa ggcggcacaa ccccagtgcc acgttgtgag ttggatagtt 1920

gtggaaagag tcaaatggct ctcctcaagc gtattcaaca aggggctgaa ggatgcccag 1980

aaggtacccc attgtatggg atctgatctg gggcctcggt gcacatgctt tacatgtgtt 2040

tagtcgaggt taaaaaacgt ctaggccccc cgaaccacgg ggacgtggtt ttcctttgaa 2100

aaacacgata atacc atg aat tcc gac ctc atg ggg tac ata ccg ctc gtc 2151
Met Asn Ser Asp Leu Met Gly Tyr Ile Pro Leu Val
1               5                   10

ggc gcc cct ctt gga ggc gct gcc agg gcc ctg gcg cat ggc gtc cgg 2199
Gly Ala Pro Leu Gly Gly Ala Ala Arg Ala Leu Ala His Gly Val Arg
        15                  20                  25

gtt ctg gaa gac ggc gtg aac tat gca aca ggg aac ctt cct ggt tgc 2247
Val Leu Glu Asp Gly Val Asn Tyr Ala Thr Gly Asn Leu Pro Gly Cys
    30                  35                  40

ttt ttc tct atc ttc ctt ctg gcc ctg ctc tct tgc ctg act gtg ccc 2295
Phe Phe Ser Ile Phe Leu Leu Ala Leu Leu Ser Cys Leu Thr Val Pro
45                  50                  55                  60

gct tca gcc tac caa gtg cgc aat tcc tcg ggg ctt tac cat gtc acc 2343
Ala Ser Ala Tyr Gln Val Arg Asn Ser Ser Gly Leu Tyr His Val Thr
                65                  70                  75

aat gat tgc cct aat tcg agt att gtg tac gag gcg gcc gat gcc atc 2391
Asn Asp Cys Pro Asn Ser Ser Ile Val Tyr Glu Ala Ala Asp Ala Ile
            80                  85                  90

ctg cac act ccg ggg tgt gtc cct tgc gtt cgc gag ggt aac gcc tcg 2439
Leu His Thr Pro Gly Cys Val Pro Cys Val Arg Glu Gly Asn Ala Ser
        95                  100                 105

agg tgt tgg gtg gcg gtg acc ccc acg gtg gcc acc agg gac ggc aaa 2487
Arg Cys Trp Val Ala Val Thr Pro Thr Val Ala Thr Arg Asp Gly Lys
    110                 115                 120

ctc ccc aca acg cag ctt cga cgt cat atc gat ctg ctt gtc ggg agc 2535
Leu Pro Thr Thr Gln Leu Arg Arg His Ile Asp Leu Leu Val Gly Ser
125                 130                 135                 140

gcc acc ctc tgc tca gcc ctc tac gtg ggg gac ctg tgc ggg tct gtt 2583
Ala Thr Leu Cys Ser Ala Leu Tyr Val Gly Asp Leu Cys Gly Ser Val
                145                 150                 155

ttt ctt gtt ggt caa ctg ttt acc ttc tct ccc agg cgc cac tgg acg 2631
Phe Leu Val Gly Gln Leu Phe Thr Phe Ser Pro Arg Arg His Trp Thr
            160                 165                 170

acg caa agc tgc aat tgt tct atc tat ccc ggc cat ata acg ggt cat 2679
Thr Gln Ser Cys Asn Cys Ser Ile Tyr Pro Gly His Ile Thr Gly His
        175                 180                 185

cgc atg gca tgg gat atg atg atg aac tgg tcc cct acg gca gcg ttg 2727
Arg Met Ala Trp Asp Met Met Met Asn Trp Ser Pro Thr Ala Ala Leu
    190                 195                 200

gtg gta gct cag ctg ctc cgg atc cca caa gcc atc atg gac atg atc 2775
Val Val Ala Gln Leu Leu Arg Ile Pro Gln Ala Ile Met Asp Met Ile
205                 210                 215                 220

gct ggt gct cac tgg gga gtc ctg gcg ggc ata gcg tat ttc tcc atg 2823
Ala Gly Ala His Trp Gly Val Leu Ala Gly Ile Ala Tyr Phe Ser Met
                225                 230                 235

gtg ggg aac tgg gcg aag gtc ctg gta gtg ctg ctg tta ttt gcc ggc 2871
Val Gly Asn Trp Ala Lys Val Leu Val Val Leu Leu Leu Phe Ala Gly
            240                 245                 250

gtc gac gcg taggatccgt cgaggaattc actcctcagg tgcaggctgc 2920
Val Asp Ala
        255

ctatcagaag gtggtggctg gtgtggccaa tgccctggct cacaaatacc actgagatct 2980

ttttccctct gccaaaaatt atggggacat catgaagccc cttgagcatc tgacttctgg 3040

ctaataaagg aaatttattt tcattgcaat agtgtgttgg aattttttgt gtctctcact 3100

cggaaggaca tatgggaggg caaatcattt aaaacatcag aatgagtatt tggtttagag 3160

tttggcaaca tatgcccata tgctggctgc catgaacaaa ggttggctat aaagaggtca 3220

tcagtatatg aaacagcccc ctgctgtcca ttccttattc catagaaaag ccttgacttg 3280

aggttagatt ttttttatat tttgttttgt gttatttttt tctttaacat ccctaaaatt 3340

ttccttacat gttttactag ccagattttt cctcctctcc tgactactcc cagtcatagc 3400

tgtccctctt ctcttatgga gatccctcga cggatcggcc gcaattcgta atcatgtcat 3460

agctgtttcc tgtgtgaaat tgttatccgc tcacaattcc acacaacata cgagccggaa 3520

gcataaagtg taaagcctgg ggtgcctaat gagtgagcta actcacatta attgcgttgc 3580

gctcactgcc cgctttccag tcgggaaacc tgtcgtgcca gctgcattaa tgaatcggcc 3640

aacgcgcggg gagaggcggt ttgcgtattg ggcgctcttc cgcttcctcg ctcactgact 3700

cgctgcgctc ggtcgttcgg ctgcggcgag cggtatcagc tcactcaaag gcggtaatac 3760

ggttatccac agaatcaggg gataacgcag gaaagaacat gtgagcaaaa ggccagcaaa 3820

aggccaggaa ccgtaaaaag gccgcgttgc tggcgttttt ccataggctc cgcccccctg 3880

acgagcatca caaaaatcga cgctcaagtc agaggtggcg aaacccgaca ggactataaa 3940

gataccaggc gtttccccct ggaagctccc tcgtgcgctc tcctgttccg accctgccgc 4000

ttaccggata cctgtccgcc tttctccctt cgggaagcgt ggcgctttct catagctcac 4060

gctgtaggta tctcagttcg gtgtaggtcg ttcgctccaa gctgggctgt gtgcacgaac 4120

cccccgttca gcccgaccgc tgcgccttat ccggtaacta tcgtcttgag tccaacccgg 4180

taagacacga cttatcgcca ctggcagcag ccactggtaa caggattagc agagcgaggt 4240

atgtaggcgg tgctacagag ttcttgaagt ggtggcctaa ctacggctac actagaagaa 4300

cagtatttgg tatctgcgct ctgctgaagc cagttacctt cggaaaaaga gttggtagct 4360

cttgatccgg caaacaaacc accgctggta gcggtggttt ttttgtttgc aagcagcaga 4420

ttacgcgcag aaaaaaagga tctcaagaag atcctttgat cttttctacg gggtctgacg 4480

ctcagtggaa cgaaaactca cgttaaggga ttttggtcat gagattatca aaaaggatct 4540

tcacctagat ccttttaaat taaaaatgaa gttttaaatc aatctaaagt atatatgagt 4600

aaacttggtc tgacagttac caatgcttaa tcagtgaggc acctatctca gcgatctgtc 4660

tatttcgttc atccatagtt gcctgactcc ccgtcgtgta gataactacg atacgggagg 4720

gcttaccatc tggccccagt gctgcaatga taccgcgaga cccacgctca ccggctccag 4780

atttatcagc aataaaccag ccagccggaa gggccgagcg cagaagtggt cctgcaactt 4840

tatccgcctc catccagtct attaattgtt gccgggaagc tagagtaagt agttcgccag 4900

ttaatagttt gcgcaacgtt gttgccattg ctacaggcat cgtggtgtca cgctcgtcgt 4960

ttggtatggc ttcattcagc tccggttccc aacgatcaag gcgagttaca tgatccccca 5020

tgttgtgcaa aaaagcggtt agctccttcg gtcctccgat cgttgtcaga agtaagttgg 5080

ccgcagtgtt atcactcatg gttatggcag cactgcataa ttctcttact gtcatgccat 5140

ccgtaagatg cttttctgtg actggtgagt actcaaccaa gtcattctga gaatagtgta 5200

tgcggcgacc gagttgctct tgcccggcgt caatacggga taataccgcg ccacatagca 5260

agaactttaa aagtgctcat cattggaaaa cgttcttcgg ggcgaaaact ctcaaggatc 5320

ttaccgctgt tgagatccag ttcgatgtaa cccactcgtg cacccaactg atcttcagca 5380

tcttttactt tcaccagcgt ttctgggtga gcaaaaacag gaaggcaaaa tgccgcaaaa 5440

aagggaataa gggcgacacg gaaatgttga atactcatac tcttcctttt tcaatattat 5500

tgaagcattt atcagggtta ttgtctcatg agcggataca tatttgaatg tatttagaaa 5560

aataaacaaa taggggttcc gcgcacattt ccccgaaaag tgccacctaa attgtaagcg 5620

ttaatatttt gttaaaattc gcgttaaatt tttgttaaat cagctcattt tttaaccaat 5680

aggccgaaat cggcaaaatc ccttataaat caaaagaata gaccgagata gggttgagtg 5740

ttgttccagt ttggaacaag agtccactat taaagaacgt ggactccaac gtcaaagggc 5800

gaaaaaccgt ctatcagggc gatggcccac tacgtgaacc atcaccctaa tcaagttttt 5860

tggggtcgag gtgccgtaaa gcactaaatc ggaaccctaa agggagcccc cgatttagag 5920

cttgacgggg aaagccggcg aacgtggcga gaaaggaagg gaagaaagcg aaaggagcgg 5980

gcgctagggc gctggcaagt gtagcggtca cgctgcgcgt aaccaccaca cccgccgcgc 6040

ttaatgcgcc gctacagggc gcgtcccatt cgccattcag gctgcgcaac tgttgggaag 6100

ggcgatcggt gcgggcctct tcgctattac gccagctggc gaaaggggga tgtgctgcaa 6160

ggcgattaag ttgggtaacg ccagggtttt cccagtcacg acgttgtaaa acgacggcca 6220

gtgagcgcgc gtaatacgac tcactatagg gcgaattgga gctccaccgc ggtg 6274

<210> 9
<211> 255
<212> PRT
<213> Artificial Sequence
<223> Description of Artificial Sequence::
      phCMV-deltaCE1-1a plasmid

<400> 9
Met Asn Ser Asp Leu Met Gly Tyr Ile Pro Leu Val Gly Ala Pro Leu
 1               5                  10                  15

Gly Gly Ala Ala Arg Ala Leu Ala His Gly Val Arg Val Leu Glu Asp
            20                  25                  30

Gly Val Asn Tyr Ala Thr Gly Asn Leu Pro Gly Cys Phe Phe Ser Ile
        35                  40                  45

Phe Leu Leu Ala Leu Leu Ser Cys Leu Thr Val Pro Ala Ser Ala Tyr
    50                  55                  60

Gln Val Arg Asn Ser Ser Gly Leu Tyr His Val Thr Asn Asp Cys Pro
65                  70                  75                  80

Asn Ser Ser Ile Val Tyr Glu Ala Ala Asp Ala Ile Leu His Thr Pro
                85                  90                  95

Gly Cys Val Pro Cys Val Arg Glu Gly Asn Ala Ser Arg Cys Trp Val
            100                 105                 110

Ala Val Thr Pro Thr Val Ala Thr Arg Asp Gly Lys Leu Pro Thr Thr
        115                 120                 125

Gln Leu Arg Arg His Ile Asp Leu Leu Val Gly Ser Ala Thr Leu Cys
    130                 135                 140

Ser Ala Leu Tyr Val Gly Asp Leu Cys Gly Ser Val Phe Leu Val Gly
145                 150                 155                 160

Gln Leu Phe Thr Phe Ser Pro Arg Arg His Trp Thr Thr Gln Ser Cys
                165                 170                 175

Asn Cys Ser Ile Tyr Pro Gly His Ile Thr Gly His Arg Met Ala Trp
            180                 185                 190

Asp Met Met Met Asn Trp Ser Pro Thr Ala Ala Leu Val Val Ala Gln
        195                 200                 205

Leu Leu Arg Ile Pro Gln Ala Ile Met Asp Met Ile Ala Gly Ala His
    210                 215                 220

Trp Gly Val Leu Ala Gly Ile Ala Tyr Phe Ser Met Val Gly Asn Trp
225                 230                 235                 240

Ala Lys Val Leu Val Val Leu Leu Leu Phe Ala Gly Val Asp Ala
                245                 250                 255

<210> 10
<211> 7754
<212> DNA
<213> Artificial Sequence

<220>
<223> Description of Artificial Sequence:

<220>
<221> CDS
<222> (2116)..(3966)

<220>
<221> promoter
<222> (1)..(768)
<223> human cytomegalovirus (hCMV) immediate-early promoter

<220>
<221> misc_feature
<222> (769)..(1429)
<223> rabbit beta-globin intron II

<220>
<221> misc_feature
<222> (1425)..(1433)
<223> BamHI/ClaI junction after ligation of pTM-E1E2-745bit (ClaI-blunted/StuI
in BamHI-blunted of hCMV-G

<220>
<221> misc_feature
<222> (1430)..(2115)
<223> IRES sequence of EMCV

<220>
<221> misc_feature
<222> (2125)..(2304)
<223> deltaC part of the polyprotein

<220>
<221> misc_feature
<222> (2305)..(2880)
<223> Mature E1 protein

<220>
<221> misc_feature
<222> (2881)..(3966)
<223> Mature E2 protein

<220>
<221> misc_feature
<222> (3979)..(4354)
<223> Sinbis Virus junk sequence

<220>
<221> misc_feature
<222> (4361)..(4368)
<223> StuI/BamHI junction after ligation of pTM-E1E2-745bit (ClaI-blunted/StuI)
in BamHI-blunted of hCMV-G


<400> 10
gcggccgctc tagagagctt ggcccattgc atacgttgta tccatatcat aatatgtaca 60

tttatattgg ctcatgtcca acattaccgc catgttgaca ttgattattg actagttatt 120

aatagtaatc aattacgggg tcattagttc atagcccata tatggagttc cgcgttacat 180

aacttacggt aaatggcccg cctggctgac cgcccaacga cccccgccca ttgacgtcaa 240

taatgacgta tgttcccata gtaacgccaa tagggacttt ccattgacgt caatgggtgg 300

agtatttacg gtaaactgcc cacttggcag tacatcaagt gtatcatatg ccaagtacgc 360

cccctattga cgtcaatgac ggtaaatggc ccgcctggca ttatgcccag tacatgacct 420

tatgggactt tcctacttgg cagtacatct acgtattagt catcgctatt accatggtga 480

tgcggttttg gcagtacatc aatgggcgtg gatagcggtt tgactcacgg ggatttccaa 540

gtctccaccc cattgacgtc aatgggagtt tgttttggca ccaaaatcaa cgggactttc 600

caaaatgtcg taacaactcc gccccattga cgcaaatggg cggtaggcgt gtacggtggg 660

aggtctatat aagcagagct cgtttagtga accgtcagat cgcctggaga cgccatccac 720

gctgttttga cctccataga agacaccggg accgatccag cctccggtcg accgatcctg 780

agaacttcag ggtgagtttg gggacccttg attgttcttt ctttttcgct attgtaaaat 840

tcatgttata tggagggggc aaagttttca gggtgttgtt tagaatggga agatgtccct 900

tgtatcacca tggaccctca tgataatttt gtttctttca ctttctactc tgttgacaac 960

cattgtctcc tcttattttc ttttcatttt ctgtaacttt ttcgttaaac tttagcttgc 1020

atttgtaacg aatttttaaa ttcacttttg tttatttgtc agattgtaag tactttctct 1080

aatcactttt ttttcaaggc aatcagggta tattatattg tacttcagca cagttttaga 1140

gaacaattgt tataattaaa tgataaggta gaatatttct gcatataaat tctggctggc 1200

gtggaaatat tcttattggt agaaacaact acaccctggt catcatcctg cctttctctt 1260

tatggttaca atgatataca ctgtttgaga tgaggataaa atactctgag tccaaaccgg 1320

gcccctctgc taaccatgtt catgccttct tctctttcct acagctcctg ggcaacgtgc 1380

tggttgttgt gctgtctcat cattttggca aagaattcct cgacggatcc gatgaaggac 1440

agttctttcc agacattgtt gaattgatct cgatcccgcg aaattaatac gactcactat 1500

agggagacca caacggtttc cctctagcgg gatcaattcc gcccctctcc ctcccccccc 1560

cctaacgtta ctggccgaag ccgcttggaa taaggccggt gtgcgtttgt ctatatgtta 1620

ttttccacca tattgccgtc ttttggcaat gtgagggccc ggaaacctgg ccctgtcttc 1680

ttgacgagca ttcctagggg tctttcccct ctcgccaaag gaatgcaagg tctgttgaat 1740

gtcgtgaagg aagcagttcc tctggaagct tcttgaagac aaacaacgtc tgtagcgacc 1800

ctttgcaggc agcggaaccc cccacctggc gacaggtgcc tctgcggcca aaagccacgt 1860

gtataagata cacctgcaaa ggcggcacaa ccccagtgcc acgttgtgag ttggatagtt 1920

gtggaaagag tcaaatggct ctcctcaagc gtattcaaca aggggctgaa ggatgcccag 1980

aaggtacccc attgtatggg atctgatctg gggcctcggt gcacatgctt tacatgtgtt 2040

tagtcgaggt taaaaaacgt ctaggccccc cgaaccacgg ggacgtggtt ttcctttgaa 2100

aaacacgata atacc atg aat tcc gac ctc atg ggg tac ata ccg ctc gtc 2151
Met Asn Ser Asp Leu Met Gly Tyr Ile Pro Leu Val
1               5                   10

ggc gcc cct ctt gga ggc gct gcc agg gcc ctg gcg cat ggc gtc cgg 2199
Gly Ala Pro Leu Gly Gly Ala Ala Arg Ala Leu Ala His Gly Val Arg
        15                  20                  25

gtt ctg gaa gac ggc gtg aac tat gca aca ggg aac ctt cct ggt tgc 2247
Val Leu Glu Asp Gly Val Asn Tyr Ala Thr Gly Asn Leu Pro Gly Cys
    30                  35                  40

ttt ttc tct atc ttc ctt ctg gcc ctg ctc tct tgc ctg act gtg ccc 2295
Phe Phe Ser Ile Phe Leu Leu Ala Leu Leu Ser Cys Leu Thr Val Pro
45                  50                  55                  60

gct tca gcc tac caa gtg cgc aat tcc tcg ggg ctt tac cat gtc acc 2343
Ala Ser Ala Tyr Gln Val Arg Asn Ser Ser Gly Leu Tyr His Val Thr
                65                  70                  75

aat gat tgc cct aat tcg agt att gtg tac gag gcg gcc gat gcc atc 2391
Asn Asp Cys Pro Asn Ser Ser Ile Val Tyr Glu Ala Ala Asp Ala Ile
            80                  85                  90

ctg cac act ccg ggg tgt gtc cct tgc gtt cgc gag ggt aac gcc tcg 2439
Leu His Thr Pro Gly Cys Val Pro Cys Val Arg Glu Gly Asn Ala Ser
        95                  100                 105

agg tgt tgg gtg gcg gtg acc ccc acg gtg gcc acc agg gac ggc aaa 2487
Arg Cys Trp Val Ala Val Thr Pro Thr Val Ala Thr Arg Asp Gly Lys
    110                 115                 120

ctc ccc aca acg cag ctt cga cgt cat atc gat ctg ctt gtc ggg agc 2535
Leu Pro Thr Thr Gln Leu Arg Arg His Ile Asp Leu Leu Val Gly Ser
125                 130                 135                 140

gcc acc ctc tgc tca gcc ctc tac gtg ggg gac ctg tgc ggg tct gtt 2583
Ala Thr Leu Cys Ser Ala Leu Tyr Val Gly Asp Leu Cys Gly Ser Val
                145                 150                 155

ttt ctt gtt ggt caa ctg ttt acc ttc tct ccc agg cgc cac tgg acg 2631
Phe Leu Val Gly Gln Leu Phe Thr Phe Ser Pro Arg Arg His Trp Thr
            160                 165                 170

acg caa agc tgc aat tgt tct atc tat ccc ggc cat ata acg ggt cat 2679
Thr Gln Ser Cys Asn Cys Ser Ile Tyr Pro Gly His Ile Thr Gly His
        175                 180                 185

cgc atg gca tgg gat atg atg atg aac tgg tcc cct acg gca gcg ttg 2727
Arg Met Ala Trp Asp Met Met Met Asn Trp Ser Pro Thr Ala Ala Leu
    190                 195                 200

gtg gta gct cag ctg ctc cgg atc cca caa gcc atc atg gac atg atc 2775
Val Val Ala Gln Leu Leu Arg Ile Pro Gln Ala Ile Met Asp Met Ile
205                 210                 215                 220

gct ggt gct cac tgg gga gtc ctg gcg ggc ata gcg tat ttc tcc atg 2823
Ala Gly Ala His Trp Gly Val Leu Ala Gly Ile Ala Tyr Phe Ser Met
                225                 230                 235

gtg ggg aac tgg gcg aag gtc ctg gta gtg ctg ctg tta ttt gcc ggc 2871
Val Gly Asn Trp Ala Lys Val Leu Val Val Leu Leu Leu Phe Ala Gly
            240                 245                 250

gtc gac gcg gaa acc cac gtc acc ggg gga agt gcc ggc cac acc acg 2919
Val Asp Ala Glu Thr His Val Thr Gly Gly Ser Ala Gly His Thr Thr
        255                 260                 265

gct ggg ctt gtt ggt ctc ctt aca cca ggc gcc aag cag aac atc caa 2967
Ala Gly Leu Val Gly Leu Leu Thr Pro Gly Ala Lys Gln Asn Ile Gln
    270                 275                 280

ctg atc aac acc aac ggc agt tgg cac atc aat agc acg gcc ttg aac 3015
Leu Ile Asn Thr Asn Gly Ser Trp His Ile Asn Ser Thr Ala Leu Asn
285                 290                 295                 300

tgc aac gat agc ctt acc acc ggc tgg tta gca ggg ctc ttc tat cgc 3063
Cys Asn Asp Ser Leu Thr Thr Gly Trp Leu Ala Gly Leu Phe Tyr Arg
                305                 310                 315

cac aaa ttc aac tct tca ggc tgt cct gag agg ttg gcc agc tgc cga    3111
His Lys Phe Asn Ser Ser Gly Cys Pro Glu Arg Leu Ala Ser Cys Arg
            320                 325                 330

cgc ctt acc gat ttt gcc cag ggc tgg ggt ccc atc agt tat gcc aac    3159
Arg Leu Thr Asp Phe Ala Gln Gly Trp Gly Pro Ile Ser Tyr Ala Asn
        335                 340                 345

gga agc ggc ctt gac gaa cgc ccc tac tgt tgg cac tac cct cca aga    3207
Gly Ser Gly Leu Asp Glu Arg Pro Tyr Cys Trp His Tyr Pro Pro Arg
    350                 355                 360

cct tgt ggc att gtg ccc gca aag agc gtg tgt ggc ccg gta tat tgc    3255
Pro Cys Gly Ile Val Pro Ala Lys Ser Val Cys Gly Pro Val Tyr Cys
365                 370                 375                 380

ttc act ccc agc ccc gtg gtg gtg gga acg acc gac agg tcg ggc gcg    3303
Phe Thr Pro Ser Pro Val Val Val Gly Thr Thr Asp Arg Ser Gly Ala
                385                 390                 395

cct acc tac agc tgg ggt gca aat gat acg gat gtc ttc gtc ctt aac    3351
Pro Thr Tyr Ser Trp Gly Ala Asn Asp Thr Asp Val Phe Val Leu Asn
            400                 405                 410

aac acc agg cca ccg ctg ggc aat tgg ttc ggt tgt acc tgg atg aac    3399
Asn Thr Arg Pro Pro Leu Gly Asn Trp Phe Gly Cys Thr Trp Met Asn
        415                 420                 425

tca act gga ttc acc aaa gtg tgc gga gcg ccc cct tgt gtc atc gga    3447
Ser Thr Gly Phe Thr Lys Val Cys Gly Ala Pro Pro Cys Val Ile Gly
    430                 435                 440

ggg gtg ggc aac aac acc ttg ctc tgc ccc act gat tgc ttc cgc aaa    3495
Gly Val Gly Asn Asn Thr Leu Leu Cys Pro Thr Asp Cys Phe Arg Lys
445                 450                 455                 460

cat ccg gaa gcc aca tac tct cgg tgc ggc tcc ggt ccc tgg att aca    3543
His Pro Glu Ala Thr Tyr Ser Arg Cys Gly Ser Gly Pro Trp Ile Thr
                465                 470                 475

ccc agg tgc atg gtc gac tac ccg tat agg ctt tgg cac tat cct tgt    3591
Pro Arg Cys Met Val Asp Tyr Pro Tyr Arg Leu Trp His Tyr Pro Cys
            480                 485                 490

act atc aat tac acc ata ttc aaa gtc agg atg tac gtg gga ggg gtc    3639
Thr Ile Asn Tyr Thr Ile Phe Lys Val Arg Met Tyr Val Gly Gly Val
        495                 500                 505

gag cac agg ctg gaa gcg gcc tgc aac tgg acg cgg ggc gaa cgc tgt    3687
Glu His Arg Leu Glu Ala Ala Cys Asn Trp Thr Arg Gly Glu Arg Cys
    510                 515                 520

gat ctg gaa gac agg gac agg tcc gag ctc agc cca ttg ctg ctg tcc    3735
Asp Leu Glu Asp Arg Asp Arg Ser Glu Leu Ser Pro Leu Leu Leu Ser
525                 530                 535                 540

acc aca cag tgg cag gtc ctt ccg tgt tct ttc acg acc ctg cca gcc    3783
Thr Thr Gln Trp Gln Val Leu Pro Cys Ser Phe Thr Thr Leu Pro Ala
                545                 550                 555

ttg tcc acc ggc ctc atc cac ctc cac cag aac att gtg gac gtg cag    3831

Leu Ser Thr Gly Leu Ile His Leu His Gln Asn Ile Val Asp Val Gln
            560                 565                 570

tac ttg tac ggg gtg ggg tca agc atc gcg tcc tgg gcc att aag tgg    3879
Tyr Leu Tyr Gly Val Gly Ser Ser Ile Ala Ser Trp Ala Ile Lys Trp
        575                 580                 585

gag tac gtc gtt ctc ctg ttc ctt ctg ctt gca gac gcg cgc gtc tgc    3927
Glu Tyr Val Val Leu Leu Phe Leu Leu Leu Ala Asp Ala Arg Val Cys
    590                 595                 600

tcc tgc ttg tgg atg atg tta ctc ata tcc caa gcg gag taagctcctt     3976
Ser Cys Leu Trp Met Met Leu Leu Ile Ser Gln Ala Glu
605                 610                 615

gggcccaatg atccgaccag caaaactcga tgtacttccg aggaactgat gtgcataatg 4036

catcaggctg gtacattaga tccccgctta ccgcgggcaa tatagcaaca ctaaaaactc 4096

gatgtacttc cgaggaagcg cagtgcataa tgctgcgcag tgttgccaca taaccactat 4156

attaaccatt tatctagcgg acgccaaaaa ctcaatgtat ttctgaggaa gcgtggtgca 4216

taatgccacg cagcgtctgc ataactttta ttatttcttt tattaatcaa caaaattttg 4276

tttttaacat ttcaaaaaaa aaaaaaaaaa aaaaaaaaaa aaaaaaaaaa gggaattcct 4336

cgattaatta agcggccgct cgagagggat ccgtcgagga attcactcct caggtgcagg 4396

ctgcctatca gaaggtggtg gctggtgtgg ccaatgccct ggctcacaaa taccactgag 4456

atctttttcc ctctgccaaa aattatgggg acatcatgaa gccccttgag catctgactt 4516

ctggctaata aaggaaattt attttcattg caatagtgtg ttggaatttt ttgtgtctct 4576

cactcggaag gacatatggg agggcaaatc atttaaaaca tcagaatgag tatttggttt 4636

agagtttggc aacatatgcc catatgctgg ctgccatgaa caaaggttgg ctataaagag 4696

gtcatcagta tatgaaacag ccccctgctg tccattcctt attccataga aaagccttga 4756

cttgaggtta gatttttttt atattttgtt ttgtgttatt tttttcttta acatccctaa 4816

aattttcctt acatgtttta ctagccagat ttttcctcct ctcctgacta ctcccagtca 4876

tagctgtccc tcttctctta tggagatccc tcgacggatc ggccgcaatt cgtaatcatg 4936

tcatagctgt ttcctgtgtg aaattgttat ccgctcacaa ttccacacaa catacgagcc 4996

ggaagcataa agtgtaaagc ctggggtgcc taatgagtga gctaactcac attaattgcg 5056

ttgcgctcac tgcccgcttt ccagtcggga aacctgtcgt gccagctgca ttaatgaatc 5116

ggccaacgcg cggggagagg cggtttgcgt attgggcgct cttccgcttc ctcgctcact 5176

gactcgctgc gctcggtcgt tcggctgcgg cgagcggtat cagctcactc aaaggcggta 5236

atacggttat ccacagaatc aggggataac gcaggaaaga acatgtgagc aaaaggccag 5296

caaaaggcca ggaaccgtaa aaaggccgcg ttgctggcgt ttttccatag gctccgcccc 5356

cctgacgagc atcacaaaaa tcgacgctca agtcagaggt ggcgaaaccc gacaggacta 5416

taaagatacc aggcgtttcc ccctggaagc tccctcgtgc gctctcctgt tccgaccctg 5476

ccgcttaccg gatacctgtc cgcctttctc ccttcgggaa gcgtggcgct ttctcatagc 5536

tcacgctgta ggtatctcag ttcggtgtag gtcgttcgct ccaagctggg ctgtgtgcac 5596

gaaccccccg ttcagcccga ccgctgcgcc ttatccggta actatcgtct tgagtccaac 5656

ccggtaagac acgacttatc gccactggca gcagccactg gtaacaggat tagcagagcg 5716

aggtatgtag gcggtgctac agagttcttg aagtggtggc ctaactacgg ctacactaga 5776

agaacagtat ttggtatctg cgctctgctg aagccagtta ccttcggaaa aagagttggt 5836

agctcttgat ccggcaaaca aaccaccgct ggtagcggtg gtttttttgt ttgcaagcag 5896

cagattacgc gcagaaaaaa aggatctcaa gaagatcctt tgatcttttc tacggggtct 5956

gacgctcagt ggaacgaaaa ctcacgttaa gggattttgg tcatgagatt atcaaaaagg 6016

atcttcacct agatcctttt aaattaaaaa tgaagtttta aatcaatcta aagtatatat 6076

gagtaaactt ggtctgacag ttaccaatgc ttaatcagtg aggcacctat ctcagcgatc 6136

tgtctatttc gttcatccat agttgcctga ctccccgtcg tgtagataac tacgatacgg 6196

gagggcttac catctggccc cagtgctgca atgataccgc gagacccacg ctcaccggct 6256

ccagatttat cagcaataaa ccagccagcc ggaagggccg agcgcagaag tggtcctgca 6316

actttatccg cctccatcca gtctattaat tgttgccggg aagctagagt aagtagttcg 6376

ccagttaata gtttgcgcaa cgttgttgcc attgctacag gcatcgtggt gtcacgctcg 6436

tcgtttggta tggcttcatt cagctccggt tcccaacgat caaggcgagt tacatgatcc 6496

cccatgttgt gcaaaaaagc ggttagctcc ttcggtcctc cgatcgttgt cagaagtaag 6556

ttggccgcag tgttatcact catggttatg gcagcactgc ataattctct tactgtcatg 6616

ccatccgtaa gatgcttttc tgtgactggt gagtactcaa ccaagtcatt ctgagaatag 6676

tgtatgcggc gaccgagttg ctcttgcccg gcgtcaatac gggataatac cgcgccacat 6736

agcagaactt taaaagtgct catcattgga aaacgttctt cggggcgaaa actctcaagg 6796

atcttaccgc tgttgagatc cagttcgatg taacccactc gtgcacccaa ctgatcttca 6856

gcatctttta ctttcaccag cgtttctggg tgagcaaaaa caggaaggca aaatgccgca 6916

aaaaagggaa taagggcgac acggaaatgt tgaatactca tactcttcct ttttcaatat 6976

tattgaagca tttatcaggg ttattgtctc atgagcggat acatatttga atgtatttag 7036

aaaaataaac aaataggggt tccgcgcaca tttccccgaa aagtgccacc taaattgtaa 7096

gcgttaatat tttgttaaaa ttcgcgttaa atttttgtta aatcagctca ttttttaacc 7156

aataggccga aatcggcaaa atcccttata aatcaaaaga atagaccgag atagggttga 7216

agtgttgttc cagtttggaa caagagtcca ctattaaaga acgtggactc caacgtcaaa 7276

gggcgaaaaa ccgtctatca gggcgatggc ccactacgtg aaccatcacc ctaatcaagt 7336

tttttggggt cgaggtgccg taaagcacta aatcggaacc ctaaagggag cccccgattt 7396

agagcttgac ggggaaagcc ggcgaacgtg gcgagaaagg aagggaagaa agcgaaagga 7456

gcgggcgcta gggcgctggc aagtgtagcg gtcacgctgc gcgtaaccac cacacccgcc 7516

gcgcttaatg cgccgctaca gggcgcgtcc cattcgccat tcaggctgcg caactgttgg 7576

gaagggcgat cggtgcgggc ctcttcgcta ttacgccagc tggcgaaagg gggatgtgct 7636

gcaaggcgat taagttgggt aacgccaggg ttttcccagt cacgacgttg taaaacgacg 7696

gccagtgagc gcgcgtaata cgactcacta tagggcgaat tggagctcca ccgcggtg 7754

<210> 11
<211> 617
<212> PRT
<213> Artificial Sequence
<223> Description of Artificial Sequence:
      phCMV-deltaCE1E2-1a plasmid

<400> 11
Met Asn Ser Asp Leu Met Gly Tyr Ile Pro Leu Val Gly Ala Pro Leu
1               5                   10                  15

Gly Gly Ala Ala Arg Ala Leu Ala His Gly Val Arg Val Leu Glu Asp
            20                  25                  30

Gly Val Asn Tyr Ala Thr Gly Asn Leu Pro Gly Cys Phe Phe Ser Ile
        35                  40                  45

Phe Leu Leu Ala Leu Leu Ser Cys Leu Thr Val Pro Ala Ser Ala Tyr
    50                  55                  60

Gln Val Arg Asn Ser Ser Gly Leu Tyr His Val Thr Asn Asp Cys Pro
65                  70                  75                  80

Asn Ser Ser Ile Val Tyr Glu Ala Ala Asp Ala Ile Leu His Thr Pro
                85                  90                  95

Gly Cys Val Pro Cys Val Arg Glu Gly Asn Ala Ser Arg Cys Trp Val
            100                 105                 110

Ala Val Thr Pro Thr Val Ala Thr Arg Asp Gly Lys Leu Pro Thr Thr
        115                 120                 125

Gln Leu Arg Arg His Ile Asp Leu Leu Val Gly Ser Ala Thr Leu Cys
    130                 135                 140

Ser Ala Leu Tyr Val Gly Asp Leu Cys Gly Ser Val Phe Leu Val Gly
145                 150                 155                 160

Gln Leu Phe Thr Phe Ser Pro Arg Arg His Trp Thr Thr Gln Ser Cys
                165                 170                 175

Asn Cys Ser Ile Tyr Pro Gly His Ile Thr Gly His Arg Met Ala Trp
            180                 185                 190

Asp Met Met Met Asn Trp Ser Pro Thr Ala Ala Leu Val Val Ala Gln
        195                 200                 205

Leu Leu Arg Ile Pro Gln Ala Ile Met Asp Met Ile Ala Gly Ala His
    210                 215                 220

Trp Gly Val Leu Ala Gly Ile Ala Tyr Phe Ser Met Val Gly Asn Trp
225                 230                 235                 240

Ala Lys Val Leu Val Val Leu Leu Leu Phe Ala Gly Val Asp Ala Glu
                245                 250                 255

Thr His Val Thr Gly Gly Ser Ala Gly His Thr Thr Ala Gly Leu Val
            260                 265                 270

Gly Leu Leu Thr Pro Gly Ala Lys Gln Asn Ile Gln Leu Ile Asn Thr
        275                 280                 285

Asn Gly Ser Trp His Ile Asn Ser Thr Ala Leu Asn Cys Asn Asp Ser
    290                 295                 300

Leu Thr Thr Gly Trp Leu Ala Gly Leu Phe Tyr Arg His Lys Phe Asn
305                 310                 315                 320

Ser Ser Gly Cys Pro Glu Arg Leu Ala Ser Cys Arg Arg Leu Thr Asp
                325                 330                 335

Phe Ala Gln Gly Trp Gly Pro Ile Ser Tyr Ala Asn Gly Ser Gly Leu
            340                 345                 350

Asp Glu Arg Pro Tyr Cys Trp His Tyr Pro Pro Arg Pro Cys Gly Ile
        355                 360                 365

Val Pro Ala Lys Ser Val Cys Gly Pro Val Tyr Cys Phe Thr Pro Ser
    370                 375                 380

Pro Val Val Val Gly Thr Thr Asp Arg Ser Gly Ala Pro Thr Tyr Ser
385                 390                 395                 400

Trp Gly Ala Asn Asp Thr Asp Val Phe Val Leu Asn Asn Thr Arg Pro
                405                 410                 415

Pro Leu Gly Asn Trp Phe Gly Cys Thr Trp Met Asn Ser Thr Gly Phe
            420                 425                 430

Thr Lys Val Cys Gly Ala Pro Pro Cys Val Ile Gly Gly Val Gly Asn
        435                 440                 445

Asn Thr Leu Leu Cys Pro Thr Asp Cys Phe Arg Lys His Pro Glu Ala
    450                 455                 460

Thr Tyr Ser Arg Cys Gly Ser Gly Pro Trp Ile Thr Pro Arg Cys Met
465                 470                 475                 480

Val Asp Tyr Pro Tyr Arg Leu Trp His Tyr Pro Cys Thr Ile Asn Tyr
                485                 490                 495

Thr Ile Phe Lys Val Arg Met Tyr Val Gly Gly Val Glu His Arg Leu

500                 505                 510

Glu Ala Ala Cys Asn Trp Thr Arg Gly Glu Arg Cys Asp Leu Glu Asp
        515                 520                 525

Arg Asp Arg Ser Glu Leu Ser Pro Leu Leu Leu Ser Thr Thr Gln Trp
    530                 535                 540

Gln Val Leu Pro Cys Ser Phe Thr Thr Leu Pro Ala Leu Ser Thr Gly
545                 550                 555                 560

Leu Ile His Leu His Gln Asn Ile Val Asp Val Gln Tyr Leu Tyr Gly
                565                 570                 575

Val Gly Ser Ser Ile Ala Ser Trp Ala Ile Lys Trp Glu Tyr Val Val
            580                 585                 590

Leu Leu Phe Leu Leu Leu Ala Asp Ala Arg Val Cys Ser Cys Leu Trp
        595                 600                 605

Met Met Leu Leu Ile Ser Gln Ala Glu
    610                 615

<210> 12
<211> 7178
<212> DNA
<213> Artificial Sequence

<220>
<223> Description of Artificial Sequence:
      phCMV-deltaCE2-1a plasmid

<220>
<221> CDS
<222> (2116)..(3390)

<220>
<221> promoter
<222> (1)..(768)
<223> human cytomegalovirus (hCMV) immediate-early promoter

<220>
<221> misc_feature
<222> (769)..(1429)
<223> rabbit beta-globin intron II

<220>
<221> misc_feature
<222> (1425)..(1430)
<223> BamHI 5'junction after ligation of deltaCE2
      fragment in BamHI site of hCMV-G

<220>
<221> misc_feature
<222> (1430)..(2115)
<223> IRES sequence of EMCV

<220>
<221> misc_feature

<220>
<221> misc_feature
<222> (2125)..(2304)
<223> deltaC part of the polyprotein

<220>
<221> misc_feature
<222> (2305)..(3390)
<223> Mature E2 protein

<220>
<221> misc_feature
<222> (3403)..(3778)
<223> Sinbis Virus junk sequence

<220>
<221> misc_feature
<222> (3787)..(3792)
<223> BamHI 3' junction after ligation of deltaCE1
      fragment in BamHI site of hCMV-G

<400> 12
gcggccgctc tagagagctt ggcccattgc atacgttgta tccatatcat aatatgtaca 60

tttatattgg ctcatgtcca acattaccgc catgttgaca ttgattattg actagttatt 120

aatagtaatc aattacgggg tcattagttc atagcccata tatggagttc cgcgttacat 180

aacttacggt aaatggcccg cctggctgac cgcccaacga cccccgccca ttgacgtcaa 240

taatgacgta tgttcccata gtaacgccaa tagggacttt ccattgacgt caatgggtgg 300

agtatttacg gtaaactgcc cacttggcag tacatcaagt gtatcatatg ccaagtacgc 360

cccctattga cgtcaatgac ggtaaatggc ccgcctggca ttatgcccag tacatgacct 420

tatgggactt tcctacttgg cagtacatct acgtattagt catcgctatt accatggtga 480

tgcggttttg gcagtacatc aatgggcgtg gatagcggtt tgactcacgg ggatttccaa 540

gtctccaccc cattgacgtc aatgggagtt tgttttggca ccaaaatcaa cgggactttc 600

caaaatgtcg taacaactcc gccccattga cgcaaatggg cggtaggcgt gtacggtggg 660

aggtctatat aagcagagct cgtttagtga accgtcagat cgcctggaga cgccatccac 720

gctgttttga cctccataga agacaccggg accgatccag cctccggtcg accgatcctg 780

agaacttcag ggtgagtttg gggacccttg attgttcttt ctttttcgct attgtaaaat 840

tcatgttata tggagggggc aaagttttca gggtgttgtt tagaatggga agatgtccct 900

tgtatcacca tggaccctca tgataatttt gtttctttca ctttctactc tgttgacaac 960

cattgtctcc tcttattttc ttttcatttt ctgtaacttt ttcgttaaac tttagcttgc 1020

atttgtaacg aatttttaaa ttcacttttg tttatttgtc agattgtaag tactttctct 1080

aatcactttt ttttcaaggc aatcagggta tattatattg tacttcagca cagttttaga 1140

tatggttaca atgatataca ctgtttgaga tgaggataaa atactctgag tccaaaccgg 1320

gcccctctgc taaccatgtt catgccttct tctctttcct acagctcctg ggcaacgtgc 1380

tggttgttgt gctgtctcat cattttggca aagaattcct cgacggatcc gatgaaggac 1440

agttctttcc agacattgtt gaattgatct cgatcccgcg aaattaatac gactcactat 1500

agggagacca caacggtttc cctctagcgg gatcaattcc gcccctctcc ctcccccccc 1560

cctaacgtta ctggccgaag ccgcttggaa taaggccggt gtgcgtttgt ctatatgtta 1620

ttttccacca tattgccgtc ttttggcaat gtgagggccc ggaaacctgg ccctgtcttc 1680

ttgacgagca ttcctagggg tctttcccct ctcgccaaag gaatgcaagg tctgttgaat 1740

gtcgtgaagg aagcagttcc tctggaagct tcttgaagac aaacaacgtc tgtagcgacc 1800

ctttgcaggc agcggaaccc cccacctggc gacaggtgcc tctgcggcca aaagccacgt 1860

gtataagata cacctgcaaa ggcggcacaa ccccagtgcc acgttgtgag ttggatagtt 1920

gtggaaagag tcaaatggct ctcctcaagc gtattcaaca aggggctgaa ggatgcccag 1980

aaggtacccc attgtatggg atctgatctg gggcctcggt gcacatgctt tacatgtgtt 2040

tagtcgaggt taaaaaacgt ctaggccccc cgaaccacgg ggacgtggtt ttcctttgaa 2100

aaacacgata atacc atg aat tcc gac ctc atg ggg tac ata ccg ctc gtc 2151
Met Asn Ser Asp Leu Met Gly Tyr Ile Pro Leu Val
1               5                   10

ggc gcc cct ctt gga ggc gct gcc agg gcc ctg gcg cat ggc gtc cgg 2199
Gly Ala Pro Leu Gly Gly Ala Ala Arg Ala Leu Ala His Gly Val Arg
        15                  20                  25

gtt ctg gaa gac ggc gtg aac tat gca aca ggg aac ctt cct ggt tgc 2247
Val Leu Glu Asp Gly Val Asn Tyr Ala Thr Gly Asn Leu Pro Gly Cys
    30                  35                  40

ttt ttc tct atc ttc ctt ctg gcc ctg ctc tct tgc ctg act gtg ccc 2295
Phe Phe Ser Ile Phe Leu Leu Ala Leu Leu Ser Cys Leu Thr Val Pro
45                  50                  55                  60

gct tca gcc gaa acc cac gtc acc ggg gga agt gcc ggc cac acc acg 2343
Ala Ser Ala Glu Thr His Val Thr Gly Gly Ser Ala Gly His Thr Thr
                65                  70                  75

gct ggg ctt gtt ggt ctc ctt aca cca ggc gcc aag cag aac atc caa 2391
Ala Gly Leu Val Gly Leu Leu Thr Pro Gly Ala Lys Gln Asn Ile Gln
            80                  85                  90

ctg atc aac acc aac ggc agt tgg cac atc aat agc acg gcc ttg aac 2439
Leu Ile Asn Thr Asn Gly Ser Trp His Ile Asn Ser Thr Ala Leu Asn
        95                  100                 105

tgc aac gat agc ctt acc acc ggc tgg tta gca ggg ctc ttc tat cgc 2487
Cys Asn Asp Ser Leu Thr Thr Gly Trp Leu Ala Gly Leu Phe Tyr Arg
    110                 115                 120

cac aaa ttc aac tct tca ggc tgt cct gag agg ttg gcc agc tgc cga    2535
His Lys Phe Asn Ser Ser Gly Cys Pro Glu Arg Leu Ala Ser Cys Arg
125                 130                 135                 140

cgc ctt acc gat ttt gcc cag ggc tgg ggt ccc atc agt tat gcc aac    2583
Arg Leu Thr Asp Phe Ala Gln Gly Trp Gly Pro Ile Ser Tyr Ala Asn
                145                 150                 155

gga agc ggc ctt gac gaa cgc ccc tac tgt tgg cac tac cct cca aga    2631
Gly Ser Gly Leu Asp Glu Arg Pro Tyr Cys Trp His Tyr Pro Pro Arg
            160                 165                 170

cct tgt ggc att gtg ccc gca aag agc gtg tgt ggc ccg gta tat tgc    2679
Pro Cys Gly Ile Val Pro Ala Lys Ser Val Cys Gly Pro Val Tyr Cys
        175                 180                 185

ttc act ccc agc ccc gtg gtg gtg gga acg acc gac agg tcg ggc gcg    2727
Phe Thr Pro Ser Pro Val Val Val Gly Thr Thr Asp Arg Ser Gly Ala
    190                 195                 200

cct acc tac agc tgg ggt gca aat gat acg gat gtc ttc gtc ctt aac    2775
Pro Thr Tyr Ser Trp Gly Ala Asn Asp Thr Asp Val Phe Val Leu Asn
205                 210                 215                 220

aac acc agg cca ccg ctg ggc aat tgg ttc ggt tgt acc tgg atg aac    2823
Asn Thr Arg Pro Pro Leu Gly Asn Trp Phe Gly Cys Thr Trp Met Asn
                225                 230                 235

tca act gga ttc acc aaa gtg tgc gga gcg ccc cct tgt gtc atc gga    2871
Ser Thr Gly Phe Thr Lys Val Cys Gly Ala Pro Pro Cys Val Ile Gly
            240                 245                 250

ggg gtg ggc aac aac acc ttg ctc tgc ccc act gat tgc ttc cgc aaa    2919
Gly Val Gly Asn Asn Thr Leu Leu Cys Pro Thr Asp Cys Phe Arg Lys
        255                 260                 265

cat ccg gaa gcc aca tac tct cgg tgc ggc tcc ggt ccc tgg att aca    2967
His Pro Glu Ala Thr Tyr Ser Arg Cys Gly Ser Gly Pro Trp Ile Thr
    270                 275                 280

ccc agg tgc atg gtc gac tac ccg tat agg ctt tgg cac tat cct tgt    3015
Pro Arg Cys Met Val Asp Tyr Pro Tyr Arg Leu Trp His Tyr Pro Cys
285                 290                 295                 300

act atc aat tac acc ata ttc aaa gtc agg atg tac gtg gga ggg gtc    3063
Thr Ile Asn Tyr Thr Ile Phe Lys Val Arg Met Tyr Val Gly Gly Val
                305                 310                 315

gag cac agg ctg gaa gcg gcc tgc aac tgg acg cgg ggc gaa cgc tgt    3111
Glu His Arg Leu Glu Ala Ala Cys Asn Trp Thr Arg Gly Glu Arg Cys
            320                 325                 330

gat ctg gaa gac agg gac agg tcc gag ctc agc cca ttg ctg ctg tcc    3159
Asp Leu Glu Asp Arg Asp Arg Ser Glu Leu Ser Pro Leu Leu Leu Ser
        335                 340                 345

acc aca cag tgg cag gtc ctt ccg tgt tct ttc acg acc ctg cca gcc    3207
Thr Thr Gln Trp Gln Val Leu Pro Cys Ser Phe Thr Thr Leu Pro Ala
    350                 355                 360

ttg tcc acc ggc ctc atc cac ctc cac cag aac att gtg gac gtg cag    3255

Leu Ser Thr Gly Leu Ile His Leu His Gln Asn Ile Val Asp Val Gln
365                 370                 375                 380

tac ttg tac ggg gtg ggg tca agc atc gcg tcc tgg gcc att aag tgg    3303
Tyr Leu Tyr Gly Val Gly Ser Ser Ile Ala Ser Trp Ala Ile Lys Trp
                385                 390                 395

gag tac gtc gtt ctc ctg ttc ctt ctg ctt gca gac gcg cgc gtc tgc    3351
Glu Tyr Val Val Leu Leu Phe Leu Leu Leu Ala Asp Ala Arg Val Cys
            400                 405                 410

tcc tgc ttg tgg atg atg tta ctc ata tcc caa gcg gag taagctcctt    3400
Ser Cys Leu Trp Met Met Leu Leu Ile Ser Gln Ala Glu
        415                 420                 425

gggcccaatg atccgaccag caaaactcga tgtacttccg aggaactgat gtgcataatg 3460

catcaggctg gtacattaga tccccgctta ccgcgggcaa tatagcaaca ctaaaaactc 3520

gatgtacttc cgaggaagcg cagtgcataa tgctgcgcag tgttgccaca taaccactat 3580

attaaccatt tatctagcgg acgccaaaaa ctcaatgtat ttctgaggaa gcgtggtgca 3640

taatgccacg cagcgtctgc ataactttta ttatttcttt tattaatcaa caaaattttg 3700

tttttaacat ttcaaaaaaa aaaaaaaaaa aaaaaaaaaa aaaaaaaaaa gggaattcct 3760

cgattaatta agcggccgct cgagagggat ccgtcgagga attcactcct caggtgcagg 3820

ctgcctatca gaaggtggtg gctggtgtgg ccaatgccct ggctcacaaa taccactgag 3880

atctttttcc ctctgccaaa aattatgggg acatcatgaa gccccttgag catctgactt 3940

ctggctaata aaggaaattt attttcattg caatagtgtg ttggaatttt ttgtgtctct 4000

cactcggaag gacatatggg agggcaaatc atttaaaaca tcagaatgag tatttggttt 4060

agagtttggc aacatatgcc catatgctgg ctgccatgaa caaaggttgg ctataaagag 4120

gtcatcagta tatgaaacag ccccctgctg tccattcctt attccataga aaagccttga 4180

cttgaggtta gatttttttt atattttgtt ttgtgttatt tttttcttta acatccctaa 4240

aattttcctt acatgtttta ctagccagat ttttcctcct ctcctgacta ctcccagtca 4300

tagctgtccc tcttctctta tggagatccc tcgacggatc ggccgcaatt cgtaatcatg 4360

tcatagctgt ttcctgtgtg aaattgttat ccgctcacaa ttccacacaa catacgagcc 4420

ggaagcataa agtgtaaagc ctggggtgcc taatgagtga gctaactcac attaattgcg 4480

ttgcgctcac tgcccgcttt ccagtcggga aacctgtcgt gccagctgca ttaatgaatc 4540

ggccaacgcg cggggagagg cggtttgcgt attgggcgct cttccgcttc ctcgctcact 4600

gactcgctgc gctcggtcgt tcggctgcgg cgagcggtat cagctcactc aaaggcggta 4660

atacggttat ccacagaatc aggggataac gcaggaaaga acatgtgagc aaaaggccag 4720

caaaaggcca ggaaccgtaa aaaggccgcg ttgctggcgt ttttccatag gctccgcccc 4780

cctgacgagc atcacaaaaa tcgacgctca agtcagaggt ggcgaaaccc gacaggacta 4840
taaagatacc aggcgtttcc ccctggaagc tccctcgtgc gctctcctgt tccgaccctg 4900
ccgcttaccg gatacctgtc cgcctttctc ccttcgggaa gcgtggcgct ttctcatagc 4960
tcacgctgta ggtatctcag ttcggtgtag gtcgttcgct ccaagctggg ctgtgtgcac 5020
gaaccccccg ttcagcccga ccgctgcgcc ttatccggta actatcgtct tgagtccaac 5080
ccggtaagac acgacttatc gccactggca gcagccactg gtaacaggat tagcagagcg 5140
aggtatgtag gcggtgctac agagttcttg aagtggtggc ctaactacgg ctacactaga 5200
agaacagtat ttggtatctg cgctctgctg aagccagtta ccttcggaaa aagagttggt 5260
agctcttgat ccggcaaaca aaccaccgct ggtagcggtg gtttttttgt ttgcaagcag 5320
cagattacgc gcagaaaaaa aggatctcaa gaagatcctt tgatcttttc tacggggtct 5380
gacgctcagt ggaacgaaaa ctcacgttaa gggattttgg tcatgagatt atcaaaaagg 5440
atcttcacct agatcctttt aaattaaaaa tgaagtttta aatcaatcta aagtatatat 5500
gagtaaactt ggtctgacag ttaccaatgc ttaatcagtg aggcacctat ctcagcgatc 5560
tgtctatttc gttcatccat agttgcctga ctccccgtcg tgtagataac tacgatacgg 5620
gagggcttac catctggccc cagtgctgca atgataccgc gagacccacg ctcaccggct 5680
ccagatttat cagcaataaa ccagccagcc ggaagggccg agcgcagaag tggtcctgca 5740
actttatccg cctccatcca gtctattaat tgttgccggg aagctagagt aagtagttcg 5800
ccagttaata gtttgcgcaa cgttgttgcc attgctacag gcatcgtggt gtcacgctcg 5860
tcgtttggta tggcttcatt cagctccggt tcccaacgat caaggcgagt tacatgatcc 5920
cccatgttgt gcaaaaaagc ggttagctcc ttcggtcctc cgatcgttgt cagaagtaag 5980
ttggccgcag tgttatcact catggttatg gcagcactgc ataattctct tactgtcatg 6040
ccatccgtaa gatgcttttc tgtgactggt gagtactcaa ccaagtcatt ctgagaatag 6100
tgtatgcggc gaccgagttg ctcttgcccg gcgtcaatac gggataatac cgcgccacat 6160
agcagaactt taaaagtgct catcattgga aaacgttctt cggggcgaaa actctcaagg 6220
atcttaccgc tgttgagatc cagttcgatg taacccactc gtgcacccaa ctgatcttca 6280
gcatctttta ctttcaccag cgtttctggg tgagcaaaaa caggaaggca aaatgccgca 6340
aaaaagggaa taagggcgac acggaaatgt tgaatactca tactcttcct ttttcaatat 6400
tattgaagca tttatcaggg ttattgtctc atgagcggat acatatttga atgtatttag 6460
aaaaataaac aaataggggt tccgcgcaca tttccccgaa aagtgccacc taaattgtaa 6520
gcgttaatat tttgttaaaa ttcgcgttaa atttttgtta aatcagctca ttttttaacc 6580
aataggccga aatcggcaaa atcccttata aatcaaaaga atagaccgag atagggttga 6640

agtgttgttc cagtttggaa caagagtcca ctattaaaga acgtggactc caacgtcaaa 6700

gggcgaaaaa ccgtctatca gggcgatggc ccactacgtg aaccatcacc ctaatcaagt 6760

tttttggggt cgaggtgccg taaagcacta aatcggaacc ctaaagggag cccccgattt 6820

agagcttgac ggggaaagcc ggcgaacgtg gcgagaaagg aagggaagaa agcgaaagga 6880

gcgggcgcta gggcgctggc aagtgtagcg gtcacgctgc gcgtaaccac cacacccgcc 6940

gcgcttaatg cgccgctaca gggcgcgtcc cattcgccat tcaggctgcg caactgttgg 7000

gaagggcgat cggtgcgggc ctcttcgcta ttacgccagc tggcgaaagg gggatgtgct 7060

gcaaggcgat taagttgggt aacgccaggg ttttcccagt cacgacgttg taaaacgacg 7120

gccagtgagc gcgcgtaata cgactcacta tagggcgaat tggagctcca ccgcggtg 7178

<210> 13
<211> 425
<212> PRT
<213> Artificial Sequence
<223> Description of Artificial Sequence:
      phCMV-deltaCE2-1a plasmid

<400> 13
Met Asn Ser Asp Leu Met Gly Tyr Ile Pro Leu Val Gly Ala Pro Leu
1               5                   10                  15

Gly Gly Ala Ala Arg Ala Leu Ala His Gly Val Arg Val Leu Glu Asp
            20                  25                  30

Gly Val Asn Tyr Ala Thr Gly Asn Leu Pro Gly Cys Phe Phe Ser Ile
        35                  40                  45

Phe Leu Leu Ala Leu Leu Ser Cys Leu Thr Val Pro Ala Ser Ala Glu
    50                  55                  60

Thr His Val Thr Gly Gly Ser Ala Gly His Thr Thr Ala Gly Leu Val
65                  70                  75                  80

Gly Leu Leu Thr Pro Gly Ala Lys Gln Asn Ile Gln Leu Ile Asn Thr
                85                  90                  95

Asn Gly Ser Trp His Ile Asn Ser Thr Ala Leu Asn Cys Asn Asp Ser
            100                 105                 110

Leu Thr Thr Gly Trp Leu Ala Gly Leu Phe Tyr Arg His Lys Phe Asn
        115                 120                 125

Ser Ser Gly Cys Pro Glu Arg Leu Ala Ser Cys Arg Arg Leu Thr Asp
    130                 135                 140

Phe Ala Gln Gly Trp Gly Pro Ile Ser Tyr Ala Asn Gly Ser Gly Leu
145                 150                 155                 160

Asp Glu Arg Pro Tyr Cys Trp His Tyr Pro Pro Arg Pro Cys Gly Ile
                165                 170                 175

Val Pro Ala Lys Ser Val Cys Gly Pro Val Tyr Cys Phe Thr Pro Ser
                180                 185                 190

Pro Val Val Val Gly Thr Thr Asp Arg Ser Gly Ala Pro Thr Tyr Ser
            195                 200                 205

Trp Gly Ala Asn Asp Thr Asp Val Phe Val Leu Asn Asn Thr Arg Pro
    210                 215                 220

Pro Leu Gly Asn Trp Phe Gly Cys Thr Trp Met Asn Ser Thr Gly Phe
225                 230                 235                 240

Thr Lys Val Cys Gly Ala Pro Pro Cys Val Ile Gly Gly Val Gly Asn
                245                 250                 255

Asn Thr Leu Leu Cys Pro Thr Asp Cys Phe Arg Lys His Pro Glu Ala
                260                 265                 270

Thr Tyr Ser Arg Cys Gly Ser Gly Pro Trp Ile Thr Pro Arg Cys Met
            275                 280                 285

Val Asp Tyr Pro Tyr Arg Leu Trp His Tyr Pro Cys Thr Ile Asn Tyr
    290                 295                 300

Thr Ile Phe Lys Val Arg Met Tyr Val Gly Gly Val Glu His Arg Leu
305                 310                 315                 320

Glu Ala Ala Cys Asn Trp Thr Arg Gly Glu Arg Cys Asp Leu Glu Asp
                325                 330                 335

Arg Asp Arg Ser Glu Leu Ser Pro Leu Leu Leu Ser Thr Thr Gln Trp
            340                 345                 350

Gln Val Leu Pro Cys Ser Phe Thr Thr Leu Pro Ala Leu Ser Thr Gly
        355                 360                 365

Leu Ile His Leu His Gln Asn Ile Val Asp Val Gln Tyr Leu Tyr Gly
    370                 375                 380

Val Gly Ser Ser Ile Ala Ser Trp Ala Ile Lys Trp Glu Tyr Val Val
385                 390                 395                 400

Leu Leu Phe Leu Leu Leu Ala Asp Ala Arg Val Cys Ser Cys Leu Trp
                405                 410                 415

Met Met Leu Leu Ile Ser Gln Ala Glu
            420                 425